Europäisches Patentamt

European Patent Office

Office européen des brevets

(19)

(11) Publication number: **0 047 600 B1**

(12) **EUROPEAN PATENT SPECIFICATION**

(45) Date of publication of patent specification: **26.02.92**

(51) Int. Cl.⁵: **C12N 15/62**, C12N 15/71, C07K 15/00, C07C 233/00

(21) Application number: **81303824.7**

(22) Date of filing: **21.08.81**

(54) Bovine pre-growth and growth hormone.

(30) Priority: **26.08.80 US 181348**

(43) Date of publication of application:
**17.03.82 Bulletin 82/11**

(45) Publication of the grant of the patent:
**26.02.92 Bulletin 92/09**

(84) Designated Contracting States:
**AT BE CH DE FR GB IT LI LU NL SE**

(56) References cited:
**EP-A- 0 001 929**
**EP-A- 0 012 494**
**EP-A- 0 020 147**
**GB-A- 2 073 245**

**THE JOURNAL OF BIOLOGICAL CHEMISTRY,
vol.255, no.16, August 25, 1980, (US)**

**NUCLEIC ACIDS RESEARCH, vol.9, no.1, 1981,
IRL Press Ltd., (GB)**

**NATURE, vol.281, October 18, 1979, Macmillan Journals Ltd.**

**SCIENCE, vol.205, August 10, 1979**

**NATURE, vol.276, December 21/28, 1978, Macmillan Journals Ltd.**

(73) Proprietor: **THE REGENTS OF THE UNIVERSITY
OF CALIFORNIA**
**2199 Addison Street**
**Berkeley, California 94720(US)**

(72) Inventor: **Miller, Walter L.**
**2050 - 44th. Street**
**San Francisco Calif. 94116(US)**
Inventor: **Martial, Joseph A.**
**Genie Genetizue Institut de Chimie - B6**
**Sart-Tilman B-4000 par Liege(BE)**
Inventor: **Baxter, John D.**
**131 San Pablo Avenue**
**San Francisco Calif. 94127(US)**

(74) Representative: **De Minvielle-Devaux, Ian
Benedict Peter et al**
**CARPMAELS & RANSFORD 43, Bloomsbury
Square**
**London WC1A 2RA(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a DNA transfer vector containing a nucleotide sequence coding for bovine pre-growth or growth hormone and microorganisms containing the transfer vector as well as a method for making the transfer vector and a method for preparing the bovine growth hormone sequence.

In one embodiment, the DNA transfer vector comprises a deoxynucleotide sequence which codes for bovine pre-growth hormone, said deoxynucleotide sequence comprising a plus strand having the sequence:

```
5' - ATG ATG GCT GCA GGC CCC CGG ACC TCC CTG
CTC CTG GCT TTC GCC CTG CTC TGC CTG CCC TGG ACT CAG GTG
GTG GGC GCC TTC CCA GCC ATG TCC TTG TCC GGC CTG TTT GCC
AAC GCT GTG CTC CGG GCT CAG CAC CTG CAC CAG CTG GCT GCT
GAC ACC TTC AAA GAG TTT GAG CGT ACC TAC ATC CCG GAG GGA
CAG AGA TAC TCC ATC CAG AAC ACC CAG GTT GCC TTC TGC TTC
TCC GAA ACC ATC CCG GCC CCC ACG GGC AAG AAT GAG GCC CAG
CAG AAA TCA GAC TTG GAG CTG CTT CGC ATC TCA CTG CTC CTC
ATC CAG TCG TGG CTT GGG CCC CTG CAG TTT CTC AGC AGA GTC
TTC ACC AAC AGC TTG GTG TTT GGC ACC TCG GAC CGT GTC TAT
GAG AAG CTG AAG GAC CTG GAG GAA GGC ATC TTG GCC CTG ATG
CGG GAG CTG GAA GAT GGC ACC CCC CGG GCT GGG CAG ATC CTC
AAG CAG ACC TAT GAC AAA TTT GAC ACA AAC ATG CGC AGT GAC
GAC GCG CTG CTC AAG AAC TAC GGT CTG CTC TCC TGC TTC CGG
AAG GAC CTG CAT AAG ACG GAG ACG TAC CTG AGG GTC ATG AAG
TGC CGC CGC TTC GGG GAG GCC AGC TGT GCC TTC TAG- 3'
```

wherein

 A   is deoxyadenyl,
 G   is deoxyguanyl,
 C   is deoxycytosyl and
 T   is thymidyl.

In a further embodiment, the DNA transfer vector comprises a sequence coding for bovine growth hormone, said deoxynucleotide sequence comprising a plus strand having either the sequence:

```
            5' - GCC TTC CCA GCC ATG TCC TTG TCC GGC CTG
    TTT GCC AAC GCT GTG CTC CGG GCT CAG CAC CTG CAC CAG CTG
    GCT GCT GAC ACC TTC AAA GAG TTT GAG CGT ACC TAC ATC CCG
    GAG GGA CAG AGA TAC TCC ATC CAG AAC ACC CAG GTT GCC TTC
    TGC TTC TCC GAA ACC ATC CCG GCC CCC ACG GGC AAG AAT GAG
    GCC CAG CAG AAA TCA GAC TTG GAG CTG CTT CGC ATC TCA CTG
    CTC CTC ATC CAG TCG TGG CTT GGG CCC CTG CAG TTT CTC AGC
    AGA GTC TTC ACC AAC AGC TTG GTG TTT GGC ACC TCG GAC CGT
    GTC TAT GAG AAG CTG AAG GAC CTG GAG GAA GGC ATC TTG GCC
    CTG ATG CGG GAG CTG GAA GAT GGC ACC CCC CGG GCT GGG CAG
    ATC CTC AAG CAG ACC TAT GAC AAA TTT GAC ACA AAC ATG CGC
    AGT GAC GAC GCG CTG CTC AAG AAC TAC GGT CTG CTC TCC TGC
    TTC CGG AAG GAC CTG CAT AAG ACG GAG ACG TAC CTG AGG GTC
    ATG AAG TGC CGC CGC TTC GGG GAG GCC AGC TGT GCC TTC TAG
    - 3'
```

wherein

A    is deoxyadenyl,
G    is deoxyguanyl,
C    is deoxycytosyl and
T    is thymidyl.

or the sequence:

```
            5' - TTC CCA GCC ATG TCC TTG TCC GGC CTG TTT
    GCC AAC GCT GTG CTC CGG GCT CAG CAC CTG CAC CAG CTG GCT
    GCT GAC ACC TTC AAA GAG TTT GAG CGT ACC TAC ATC CCG GAG
    GGA CAG AGA TAC TCC ATC CAG AAC ACC CAG GTT GCC TTC TGC
    TTC TCC GAA ACC ATC CCG GCC CCC ACG GGC AAG AAT GAG GCC
    CAG CAG AAA TCA GAC TTG GAG CTG CTT CGC ATC TCA CTG CTC
    CTC ATC CAG TCG TGG CTT GGG CCC CTG CAG TTT CTC AGC AGA
    GTC TTC ACC AAC AGC TTG GTG TTT GGC ACC TCG GAC CGT GTC
    TAT GAG AAG CTG AAG GAC CTG GAG GAA GGC ATC TTG GCC CTG
    ATG CGG GAG CTG GAA GAT GGC ACC CCC CGG GCT GGG CAG ATC
    CTC AAG CAG ACC TAT GAC AAA TTT GAC ACA AAC ATG CGC AGT

    GAC GAC GCG CTG CTC AAG AAC TAC GGT CTG CTC TCC TGC TTC
    CGG AAG GAC CTG CAT AAG ACG GAG ACG PAC CTG AGG GTC ATG
    AAG TGC CGC CGC TTC GGG GAG GCC AGC TGT GCC TTC TAG - 3'
```

wherein

A    is deoxyadenyl,

G    is deoxyguanyl,

C    is deoxycytosyl and

T    is thymidyl.

In a still further embodiment, the transfer vector is pBP348, or a plasmid differing therefrom only in the length of the poly(A) and/or poly(C) portions thereof.

The DNA transfer vectors of the invention may be used to transform microorganisms, and the invention provides for microorganisms transformed by these transfer vectors.

The transformed microorganisms may produce a fusion protein having the amino acid sequence of either bovine pre-growth hormone or bovine growth hormone at its C-terminal end and a portion of a procaryotic protein as its N-terminal end.

The invention further provides a method for making a DNA transfer vector containing the bovine pregrowth hormone sequence characterized by reacting a deoxynucleotide sequence coding for bovine growth hormone with a DNA molecule prepared by cleaving a transfer vector with a restriction enzyme.

In one embodiment, a method for making the plasmid pBP348 containing the bovine pre-growth hormone sequence is characterized in that the DNA molecule prepared by cleaving a transfer vector with a restriction enzyme is pBR322 and the restriction enzyme is Pst I.

The invention also provides a method for making a DNA transfer vector containing the bovine growth hormone sequence characterized by reacting a deoxynucleotide sequence coding for bovine growth hormone with a DNA molecule prepared by cleaving a transfer vector with a restriction enzyme.

The invention further provides for a method for preparing an expression transfer vector characterized in that the DNA molecule prepared by cleaving a transfer vector with a restriction enzyme is cleaved at a point within an expressible control region.

The invention further provides for a method of making a fusion protein comprising the amino acid sequence of bovine pre-growth hormone or the amino acid sequence of bovine growth hormone as its C-terminal end and a portion of a procaryotic protein as its N-terminal end characterized by incubating a microorganism transformed by an expression transfer vector comprising a deoxynucleotide sequence coding for said bovine pregrowth hormone or for said bovine growth hormone.

The invention also provides for a method for synthesizing bovine growth hormone characterized by incubating a microorganism transformed by an expression transfer vector comprising a deoxynucleotide sequence coding for said bovine growth hormone under conditions suitable for expression of said sequence coding for bovine growth hormone and purifying bovine growth hormone from the lysate or culture medium of said microorganism.

Growth hormone is a polypeptide hormone synthesized in and secreted by the adenohypophysis (anterior lobe of the pituitary). Growth hormone is synthesized as a precursor protein (pre-growth hormone) containing an N-terminal signal peptide and the growth hormone sequence. The amino acid sequence for bovine growth hormone has been determined (Dayhoff, M.D., et al., Atlas of Protein Sequence and Structure, Vol. 5, Supp. 3, pp. 245-352, National Biomedical Research Foundation, Washington, D.C. (1978)-).

Growth hormone is normally produced throughout life, although in highest amounts during the pre-adult period. The hormone is required for pre-adult growth. Although its mechanism is not understood in detail, growth hormone is known to promote skeletal growth, nitrogen retention, protein synthesis and affects glucose and lipid metabolism. In other words, growth hormone is a general anabolic agent.

Uses of bovine growth hormone are based on its known biological activity described above. Bovine growth hormone may be administered to young cattle in order to increase their rate of growth and weight gain, thereby decreasing the time required between birth and marketing for beef. The resulting increase in meat production could be significant. Furthermore, bovine growth hormone differs from ovine growth hormone by only a few amino acids. Thus, bovine growth hormone may be administered to sheep to accomplish the same goal in sheep as in cattle, i.e., increasing rate of growth and weight gain and thus increasing meat production. It is also possible that bovine growth hormone can be administered to hogs or other animals to accomplish the same goals.

Basic techniques for cloning DNA sequences are now known. For example, Seeburg, P.H., et al., **Nature, 270,** 486 (1977), describes the cloning of the rat growth hormone gene; Shine, J., et al., **Nature, 270,** 494 (1977) describes the cloning of the human chorionic somatomammotropin gene; and Derynck, R., et al., **Nature, 285,** 542 (1980) describes the cloning of the human fibroblast interferon gene.

Methods for the expression of heterologous DNA in a microorganism are now known. In principle, the heterologous DNA coding sequence is inserted in a DNA transfer vector at a point located within an expressible operon. For the production of a hybrid protein the inserted sequence must be in reading frame phase with the coding sequence of the operon, and oriented in the same direction with respect to translation. When the conditions are met, translation of the operon results in "readthrough" to the inserted coding sequence such that the protein produced is a fusion protein comprising an N-terminal amino acid sequence coded by the expressible operon, followed by an amino acid sequence coded by the insert. See Polisky, B., et al., **Proc.Nat.Acad.Sci.USA, 73,** 3900 (1976); Itakura, K., et al., **Science, 198,** 1056 (1979). Several expressible operons have been employed, including those for $\beta$-galactosidase, $\beta$-lactamase, and tryptophan.

Abbreviations used herein are those abbreviations commonly accepted and used by one of ordinary skill in the art. For example, these abbreviations are accepted by the J.Biol.Cnem., without further elucidation.

## SUMMARY OF THE INVENTION

The present invention discloses the cloning of a DNA coding for bovine pre-growth hormone or bovine growth hormone and the expression of the cloned DNA in microorganisms.

mRNA coding for bovine pre-growth hormone is isolated from bovine pituitaries. A reverse transcript (a cDNA copy) of the mRNA is prepared and inserted into a transfer vector. A deoxynucleotide sequence coding for bovine growth hormone is prepared by hydrolyzing the sequence coding for the pre-sequence from the cDNA for bovine pre-growth hormone. The transfer vector is used to transform bacteria which express the cloned cDNA.

## DETAILED DESCRIPTION OF THE INVENTION

A DNA sequence coding for bovine growth hormone is obtained by using the cDNA method. The basic techniques of the cDNA method are known and can be illustrated by Seeburg, P.H., et al., supra, and Derynck, R., et al., supra. The cDNA is synthesized by using an RNA preparation extracted from bovine pituitaries as the template.

The RNA is isolated from bovine pituitaries using conventional techniques. Polyadenylated RNA is isolated by affinity chromatography. The integrity of the polyadenylated RNA is assessed by performing cell-free translation of the polyadenylated RNA as described by Miller, W.L. and McCarthy, B.J., **J.Biol.Chem., 254,** 742 (1979) and Martial, J.A., et al., **Proc.Nat.Acad. Sci.,USA, 74,** 1816 (1977) and analyzing the proteins produced by SDS-acrylamide gel electrophoresis as described by Laemmli, U.K., **Nature, 227,** 680 (1970). Bovine growth hormone is further identified by an immune precipitation reaction as described by Martial, J.A., et al., Proc.Nat.Acad.Sci.USA, supra.

The polyadenylated RNA is used as the template for preparing a double-stranded cDNA copy using conventional techniques. The first cDNA strand is synthesized using reverse transcriptase, an oligo-dT primer and the polyadenylated RNA as described by Miller and McCarthy, supra, and Monahan, J.J., et al., **Biochem., 15,** 223 (1976). RNA is removed by alkali digestion and the single-stranded cDNA is used to self-prime the synthesis of the second strand by reverse transcriptase. The single-stranded hairpin loop is removed by digestion with SI nuclease (Leong, J.A., et al., **J.Virol., 9,** 891 (1972), as described by Ullrich, A., et al., Science, 196:1313 (1977).

The cDNA is now ready for insertion into an appropriate transfer vector. Suitable transfer vectors include those which are capable of transforming bacteria, such as Escherichia coli and Bacillus subtilis, yeast (Saccharomyces cerevisiae), the inl, csp and esp mutant strains of Neurospora crassa and animal cells in tissue culture. Examples of transfer vectors suitable for use in this invention which are capable of transforming E. coli include: the plasmids pSC101, pMB9, pBR313, pBR315, pBR 316 and pBR322 and the vectors derived from bacteriophage, i.e., Charon 3A, Charon 4A, Charon 16A and $\lambda$ gtWES$\cdot\lambda$B. References which describe the preparation and characterizations of these transfer vectors are listed in Table 1.

## Table 1

| Transfer Vector | Reference(s) |
|---|---|
| pSC101 | Cohen, et al., <u>Proc.Natl.Acad.Sci. USA</u>, <u>70</u>, 1293 and 3240 (1977). |
| | Boyer, et al., <u>Recombinant Molecules</u>, Beers, et al., Eds., Raven Press, NY, at p. 13 (1977). |
| | Cohen, et al., <u>Recombinant Molecules</u>, at p. 91. |
| pMB9 | Rodriguez, et al., <u>Molecular Mechanisms in Control of Gene Expression</u> (ICN-UCLA Symposium on Mol. & Cell. Biol. Vol. 5), Nieslich, et al., Eds., Academic Press, NY, at p. 471 (1976). |
| | Bolivar, et al., <u>Gene</u>, <u>2</u>, 75 (1977). |
| pBR313 | Bolivar et al., <u>supra</u>. |
| pBR315 | Rodriguez et al., <u>supra</u>. |
| pBR316 | Rodriguez et al., <u>supra</u>. |
| <u>pBR322</u> | Bolivar et al., <u>Gene</u> <u>2</u>, 95 (1977). |
| | Bolivar et al., <u>Gene</u> <u>4</u>, 121 (1978). |
| | Sutcliffe, <u>Cold Spring Harbor Symp. on Quant. Biol.</u>, <u>43</u>, 77 (1978). |
| Charon 3A | Blattner et al., <u>Science</u> <u>196</u>, 161 (1977) |
| Charon 4A | Blattner et al., <u>supra</u>. |
| Charon 16A | Blattner et al., <u>supra</u>. |
| $\lambda$ gtWES · $\lambda$ B | Tremeier et al., <u>Nature</u> <u>263</u>, 526 (1976). |
| | Leder et al., <u>Science</u> <u>196</u>, 175 (1977). |

Other suitable vectors for transforming E. coli are described in Sinsheimer, R.L., **Ann.Rev.Biochem. 46,** 415 (1977). Suitable transfer vectors for transforming B. subtilis have been described by Iordanescu, S., **J. Bacteriol. 124,** 597 (1964) and Ehrlich, S.D., **Proc.Natl. Acad.Sci.USA 74,** 1680 (1977). One such vector has been identified as plasmid pC194. Hybrid plasmids containing yeast DNA -- plasmid and/or chromosomal -- and bacterial DNA -- e.g., plasmid -- are used to transform yeast. Such plasmids have been described by Beggs, J.D., **Nature 275,** 104 (1978); Asiav, C.L. and Carbon, J., **Proc.Natl.Acad. Sci.USA 76,** 3829 (1979); and Kingsman, A.K., et al., **Gene 7,** 141 (1979). Transfer vectors which can be utilized to transform animal cells in tissue culture include Adeno defective virus, SV-40 defective virus and polyoma virus.

The cDNA is inserted into a transfer vector using conventional techniques. The cDNA is usually inserted into any restriction site which is unique in the transfer vector. Charon 3A and 4A and λgt WES·λB do not contain unique sites and, as a result, restriction sites of limited number are used for these vectors. The unique restriction sites in various transfer vectors and the useful restriction sites in vectors Charon 3A and 4A and λgt WES·λB are shown in Table 2.

Table 2

| Transfer Vector | Unique Restriction Sites |
|---|---|
| pSC101 | EcoRI, Hind III, Sal I, Bam HI, Hpa I, Sma I |
| pMB9 | EcoRI, Hind III, Sal I, Bam HI |
| pBR313 | EcoRI, Hind III, Sal I, Bam HI, Hpa I, Sma I, Xma I |
| pBR315, pBR322 | EcoRI, Hind III, Sal I, Bam HI, Pst I |
| pBR316 | Hind III, Sal I, Bam HI, Pst I |
| pC194 | Hind III |
| Charon 16A | EcoRI, Sst I |
| Charon 3A | EcoRI |
| Charon 4A | EcoRI |
| λgt WES·λB | EcoRI |

Generally, the cDNA is inserted into a restriction site within a phenotypic trait for ease in selection. For example, Hind III, Sal I and Bam HI in pSC101, pMB9, pBR313, pBR315, pBR316, and pBR322 are within the gene for tetracycline resistance or its control region. Insertion at this site results in loss of tetracycline resistance. The cDNA can be inserted into the transfer vector by the use of restriction site linkers or deoxynucleotide tailing. In the former a synthetic nucleotide containing a recognition site for a particular restriction endonuclease, such as those discussed above, can be blunt-end ligated to the cDNA. The cDNA and transfer vector are separately incubated with the restriction endonuclease and then annealed to form the transfer vector containing the cDNA. In the latter, the cDNA can be tailed using a deoxynucleotide and terminal transferase as described by Roychoudhury, R., et al., **Nucl.Acids Res., 3,** 863 (1976). The transfer vector, after digestion with a restriction endonuclease, can be tailed using the complimentary deoxynucleotide in the same procedure. The tailed transfer vector and tailed cDNA are then annealed to form the transfer vector containing the cDNA. For example, the cDNA can be dC-tailed and the transfer vector can be opened at the Pst I site and dG-tailed.

The transfer vector containing the cDNA is then used to transform a suitable host. Hosts which are suitable for the various transfer vectors include E. coli, B. subtilis, yeast, N. crassa, and animal cells in tissue culture. The transfer vectors which are capable of transforming each of these hosts have been described above. Three mutant strains of E. coli are conventionally utilized. These are χ1776, RRI and HB101. E. coli χ1776 has been described in Curtis, R., **Ann.Rev. Microbiol. 30,** 507 (1976) and U.S. Patent No. 4,190,495. E. coli RRI has been described in Dugaiczyk, A., et al., Molecular Mechanisms in Control of Gene Expression at p. 543. E. coli HB101 has been described in Kedes, D.H. and Thomas, C.A., **Proc.Natl.Acad.Sci.USA 73,** 1537 (1976). The suitable hosts are transformed by conventional techniques. For example, E. coli χ1776 is transformed by the transfer vector containing the cDNA as described by Cooke, N.E., et al., **J.Biol.Chem., 255,** 6502 (1980). Colonies are selected and/or screened by conventional techniques. For example, colonies may be selected based on the loss of a phenotypic trait, such as tetracycline resistance. Colonies may be screened by (1) removing the cDNA by an appropriate restriction endonuclease and analyzing it by electrophoresis and hybridization (Southern E.M., **J.Mol.Biol., 98,** 503 (1975)), or (2) replica-plating as described by Grunstein, M. and Hogness, D.S., **Proc.Nat.Acad.Sci.USA, 72,** 3961 (1975) and hybridizing with an appropriate probe, or (3) examining colonies directly for expression by RIA or other techniques.

DNA coding for bovine growth hormone can be prepared from the insert coding for bovine growth prehormone. The DNA coding for the pre-hormone is removed by an appropriate restriction endonuclease. For example, if the cDNA is inserted in the Pst I site of the plasmid pBR322, the cDNA insert can be removed by partial digestion with Pst I as the cDNA for bovine GH contains two internal Pst I sites. The cDNA is then digested with Hae II. Alternatively, the recombinant plasmid derived from pBR322 can be digested with Hae II to remove a portion of the cDNA insert. The Hae II digestion removes the DNA coding for the N-terminal signal peptide and two of the three bases coding for the N-terminal Ala of growth hormone. These bases are replaced by incubating the insert with the Klenow fragment of DNA polymerase I and the deoxynucleotides. Alternatively, the bases for coding for Ala can be deleted by incubating the cDNA with Klenow fragment, T4 DNA polymerase or 3'-5' exonuclease in the presence of dATP. The Klenow fragment has been described by Klenow, H. and Henningsen, I., **Proc.Natl.Acad.Sci.USA 65,** 168 (1970). The cDNA coding for growth hormone is then inserted into an appropriate transfer vector as described above.

The cloned DHA is expressed in bacteria to yield either a fusion protein comprising the bovine growth hormone coded by the inserted sequence, or the bovine growth hormone itself. Several possible techniques are available as options, and may include (a) modification of the coding sequences to provide an exact desired translational starting point; (b) selection or construction of an optimal expression vector; (c) post-translational processing, either by exploiting in vivo processing activity of the host or by in vitro chemical means; and (d) direct expression. When a fusion protein is expressed, modification of the cloned nucleotide sequence will generally be unnecessary as long as the resulting sequence permits translation of the insert in the correct reading frame and no stop codons intervene before the initial codon of the inserted sequence.

Growth pre-hormone or growth hormone is expressed as a fusion protein by insertion of the cDNA into appropriate sites within expressed operons (expression vectors) including for example, the Pst I site in the $\beta$-lactamase gene of pBR 322 (Villa-Komaroff, L., et al., **Proc.Nat.Acad.Sci.USA, 75,** 3727 (1978); (Serburg, P., et al., Nature 274:795 (1978)), the EcoRI site of pBR322 carrying the lac control region and coding sequence for $\beta$-galactosidase (Itakura, K., supra), or the HindIII site of the trpD gene of plasmid ptrpED50 (Martial, J., et al., **Science, 205,** 602 (1979)). Modifications of sequence length by one or two nucleotides in order to achieve correct reading frame phase are well known in the art. Insertions at the Pst I site of pBR322, with the aid of the tailing procedure, occur in correct phase and reading frame with a probability of 1/6.

Growth pre-hormone or growth hormone is prepared from a fusion protein susceptible of specific cleavage in vitro. The cloned nucleotide sequence is modified to code for amino acid sequences providing specificity for a proteolytic enzyme. A useful sequence is AspAspAspAspLys, cleaved preferentially by the enzyme enterokinase, as described in copending application Serial No. 125,878, filed February 29, 1980, incorporated herein by reference. As described therein, a linking nucleotide sequence coding for the foregoing amino acid sequence is inserted adjacent the nucleotide sequence coding for the amino terminus of growth pre-hormone.

For growth pre-hormone such insertion requires modification of the original cDNA insert, by removal of nucleotides on the 5' end of the growth pre-hormone coding sequence. The cDNA insert for growth hormone, described supra, does not need to be modified. The modification of the insert for growth pre-hormone is accomplished either by controlled digestion of the 3' end of the insert using 3' exonuclease or T4 DNA polymerase or by the combination of restriction endonuclease cleavage at a point to the 5' side of the desired starting point and chemical synthesis to restore that portion of the desired sequence thus removed. For further details of these procedures, see copending U.S. application Serial No. 125,878. By following these procedures, preferably using T4 DNA polymerase and SI nuclease, the cDNA sequence coding for growth pre-hormone and lacking the 5' untranslated region is obtained. The linker nucleotide sequence coding for the foregoing amino acid sequence is blunt-end ligated to the cDNA coding for either growth pre-hormone or growth hormone using DNA ligase as described by Valenzuela et al., **Nature, 280,** 815 (1979). The modified cDNA sequence is inserted into a fusion protein expression vector as previously described. Host bacteria, such as E. coli HB101, RRI, or $\chi$1776, or other bacteria, are transformed by the recombinant vectors bearing the inserted growth pre-hormone coding region. Transformants are selected for resistance to ampicillin. Transformants are then grown under conditions suitable for expression of the fusion protein. After expression of the fusion protein, the growth pre-hormone or growth hormone is cleaved out by enzymatic hydrolysis using enterokinase.

By the use of appropriate expression transfer vectors, the growth pre-hormone of the present invention is expressed directly, i.e., not fused to any procaryotic protein. The underlying principle of direct expression is that the inserted DNA segment entirely replaces the coding segment normally transcribed and translated by the bacterial control region. The essential component of the control region to be preserved is termed the expression unit, which includes a promoter and a ribosomal binding site capable of acting in the host organism. It is not necessary to remove all of the nucleotides coding for the host portion of the fusion protein. The relationship between the ribosomal binding site and the start codon (AUG) is such that the start codon may be located anywhere within 3-11 nucleotides of the ribosomal binding site. Shine, J., et al., **Proc.Nat.Acad.Sci.USA, 71,** 1342 (1974) and Steitz, J., **Proc.Nat.Acad.Sci.USA, 72,** 4734 (1975). In this 3-11 nucleotide region, the first AUG to be encountered sets the reading frame for translation. In the case of ptrpE30, derived from ptrpED50 described, supra, and containing the operator, promoter, attenuator and ribosome binding sequences of the tryptophan operon together with the nucleotide sequence coding for seven amino acids of the trp E protein followed by a HindIII site, the removal of a minimum of 23-29 nucleotides from the HindIII site provides a site for insertion of the cDNA insert under tryptophan operon control.

For the direct expression of growth pre-hormone, the original cDNA insert is modified as described above to remove the 5' untranslated region. A vector for direct expression can be constructed by modification of ptrpE30 by removing 23-29 nucleotides using T4 DNA polymerase and SI nuclease as described above. A linker nucleotide sequence containing the restriction sequence for Bam HI endonuclease is blunt-end ligated to both the modified cDNA insert and the modified ptrpE30 by the procedure of Valenzuela, et al., supra. This is done to facilitate insertion which is performed essentially as described by Ullrich, A., et al., **Science, 196,** 1313 (1977). Suitable hosts, such as E. coli HB101, RRI, or χ1776 or other bacteria are transformed by the recombinant vectors bearing the inserted growth pre-hormone coding region. Transformants are selected for resistance to ampicillin and then grown under conditions suitable for expression of growth pre-hormone.

Growth hormone can be expressed directly by following the procedure described in Goeddel, D.V., et al., **Nature, 281,** 544 (1979). Alternatively, a linker nucleotide sequence containing the Bam HI site and the start codon (AUG) can be blunt-end ligated to the cDNA coding for growth hormone. This modified cDNA is then inserted into the modified ptrpE30 as described above.

Growth pre-hormone is converted to growth hormone by removal of the N-terminal sequence of hydrophobic amino acids that comprise the signal peptide. In vitro removal of the signal peptide might be carried out by treating the protein extracted from transformed, induced cells with a preparation of "rough" microsomes as described by Jackson, R.C. and Blobel, G., **Proc. Nat.Acad.Sci.USA, 74,** 5598 (1977). In vivo removal of the signal peptide may occur during direct bacterial expression of the growth pre-hormone coding sequence. Bacterial and mammalian signal peptides share sequence similarities. Proteins having mammalian signal peptides may be processed by bacterial cells resulting in excretion of growth hormone into the periplasmic space or into the medium.

Growth pre-hormone and growth hormone synthesized as described are purified by techniques well known in the art, including for example, gel filtration, ion exchange chromatography, affinity chromatography and differential solubility techniques.

The details of the present invention will be further described by the following examples. In these examples, digestions with restriction endonucleases were carried out under conditions optimized for each enzyme. Restriction endonucleases, their nomenclature and site specificity have been described in detail by Roberts, R., **Crit.Rev.Biochem., 4,** 123 (1976). Enzymes were obtained commercially (New England Biolabs, Cambridge, Massachusetts) and optimal conditions according to supplier's recommendations were employed unless noted otherwise. Reverse transcriptase was provided by Dr. J. Beard, Life Sciences, Inc., St. Petersburg, Florida. The use of reverse transcriptase and suitable reaction conditions have been described previously by Seeburg, P.H., et al., **Nature 276,** 795 (1978); Seeburg, P.H., et al., supra; and Shine, J., et al., supra. T4 DNA polymerase was obtained from New England Biolabs. The use of T4 DNA polymerase and suitable reaction conditions have been previously described in copending application Serial No. 125,878. Micrococcal SI nuclease was obtained from Miles Laboratories, Elkhart, Indiana. The use of SI nuclease and suitable reaction conditions have been previously described by Ullrich, A., supra. Terminal deoxynucleotide transferase was obtained from Enzo Biochemicals, New York, New York. The use of this enzyme and suitable reaction conditions have been previously described by Roychoudhury et al., supra. The Klenow fragment of DNA polymerase I was obtained from Boehringer Biochemicals, Indianapolis, Indiana.

EXAMPLE 1

Synthesis of bovine growth pre-hormone cDNA. Female bovine pituitaries were collected shortly after killing and were frozen immediately in liquid nitrogen. Total RNA was prepared by homogenizing the pituitaries in a guanidine thiocyanate solution, Chirginwin, J.M., et al., **Biochem., 18,** 5294 (1979). The RNA was centrifuged through 5.7M CsCl as described by Ullrich, A., et al., supra. The RNA was then extracted with phenol and precipitated with ethanol. Polyadenylated RNA was purified using oligo-dT-cellulose affinity chromatography as described by Miller and McCarthy, supra, and Aviv, H. and Leder, P., **Proc.Nat.Acad.Sci.USA,** 69, 1408 (1972).

The polyadenylated RNA was translated in a cell-free system using rabbit reticulocytes as described by Miller and McCarthy, supra, and Martial, J.A., et al., supra (1977). Bovine growth pre-hormone synthesized in this system was immune precipitated using a heterologous anti-ovine growth hormone antiserum and prepared by adsorption to formalin-fixed Staphylococcus aereus Cowan strain I as described by Martial, J.A., et al., supra (1977). The $^{35}$S-proteins were electrophoresed on 12.5% SDS slab polyacrylamide gels as described by Laemmli, supra. This analysis indicated that polyadenylated RNA coding for bovine growth pre-hormone represented about 12.6% of the total pituitary polyadenylated RNA.

Polyadenylated RNA was reverse-transcribed into single-stranded cDNA using reverse transcriptase by the procedure described by Miller and McCarthy, supra, and Monahan et al., supra. RNA was removed by alkaline hydrolysis. The single-stranded cDNA was extracted with phenol, chromatographed over G-50 Sephadex (trademark Pharmacia, Inc., Uppsala, Sweden) and ethanol precipitated. The single-stranded cDNA was used to self-prime the synthesis of the second strand of cDNA using reverse transcriptase as described above. The single-stranded "hairpin loop" at the 3' end of the first strand of cDNA was removed by digestion with SI nuclease as described by Leong et al., supra, and Ullrich, A. et al., supra. The double-stranded cDNA was purified by phenol extraction, chromatography over G-50 Sephadex and ethanol precipitation. The double-stranded cDNA was 3'dCMP tailed using dCTP and terminal transferase as described by Roychoudhury et al., supra.

Plasmid pBR322 was cleaved by Pst I endonuclease and tailed with dGMP by the previously described tailing procedure except that dGTP is used instead of dCTP. 50 ng of the dG-tailed, Pst I cleaved plasmid pBR322 and 20 ng of the dC-tailed double-stranded cDNA were annealed in a 50 μl reaction as described by Cook, et al., supra.

Transformation of E. coli χ1776 with the plasmid preparation was carried out as follows. E. coli χ1776 were rendered permeable to DNA by incubation in 75mM CaCl₂, 5mM MgCl₂, 10mM Tris, pH 7.5, for 20 minutes at 4°C. Plasmid and bacteria were incubated for 60 minutes at 4°C, and then 2 minutes at 41°C. Transformed colonies were selected for tetracycline resistance. The presence of cloned DNA was determined by colony hybridization to freshly prepared bovine pituitary $^{32}$P labelled cDNA as described by Grunstein and Hogness, supra. Plasmid DNA was prepared from selected colonies, cut with Pst I, electrophoresed on 1% agarose, stained with ethidium bromide and photographed and finally transferred to nitrocellulose filters by the method of Southern, supra. The presence of growth hormone sequences in the transferred DNA was assessed by hybridization to cloned full-length rat hormone cDNA (Seeburg, P. H., et al., supra), labelled by nick translation (Maniatis, R., et al., **Proc.Nat.Acad.Sci.USA, 72,** 1184 (1975)). One clone was obtained which hybridized with the nick-translated DNA and was designated pBP348. The insert contains 831 base pairs.

## EXAMPLE 2

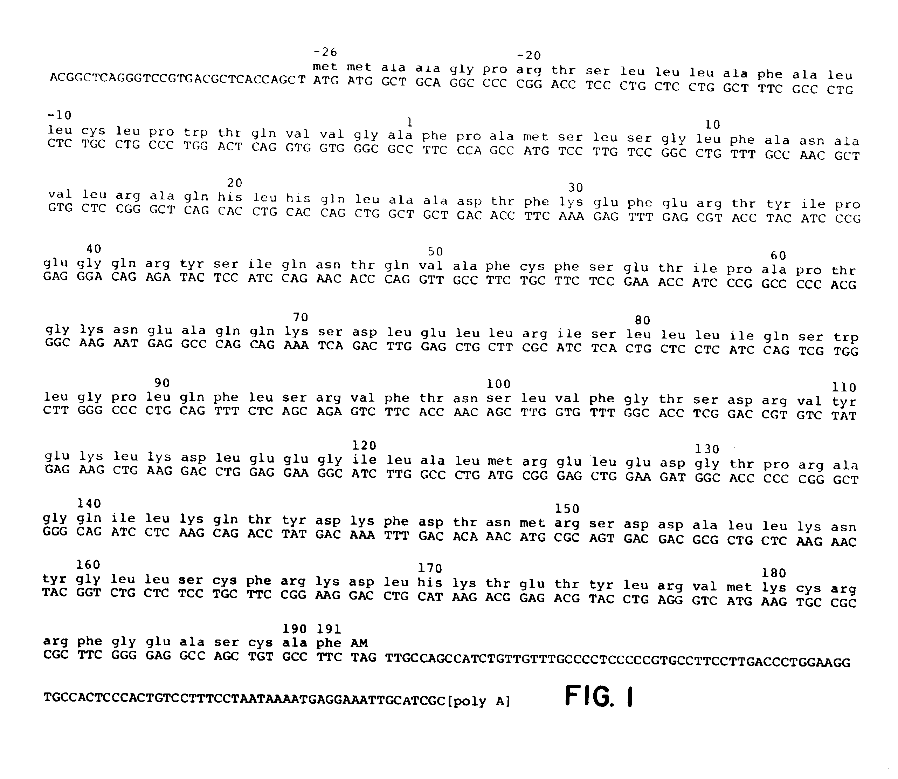

Sequence analysis of the cDNA. Plasmid pBp348 was cut with Pst I and the phosphate on the 5' ends of the DNA framents was removed with alkaline phosphatase and replaced with [$^{32}$P] phosphate using polynucleotide kinase. Subsequent cutting with a variety of other restriction endonucleases, polyacrylamide gel electrophoresis, and staining and autoradiography of the bands of DNA provide a restriction map of the cloned DNA. A large batch of pBP348 was then prepared and cut with Pst I, Pvu II or Sau 3A, labelled with [ν$^{32}$P]ATP and polynucleotide kinase, and then cut with other enzymes to yield DNA fragments labelled at a single end. These fragments were prepared by elution from polyacrylamide gel and sequenced as described by Cooke et al., supra, and Maxam, A.M. and Gilbert, W., **Proc.Nat.Acad.Sci.USA, 74,** 1560 (1977). The sequence for the inserted cDNA is shown in Figure 1 together with the corresponding predicted amino acid sequence coded by the sense strand, i.e., the strand corresponding in sequence to the respective mRNA.

The correct reading frame is recognized by the lack of termination codons over a substantial portion of the inserts. The amino acid positions are numbered beginning with the amino-terminal amino acid of bovine growth hormone and proceeding in the positive direction to the carboxy terminal end and in the negative direction to the first AUG codon presumed to be the point of translation initiation. The sequences suggest, in common with many other hormones, the synthesis of growth hormone involves posttranslational processing. The translation of growth hormone mRNA yields a precursor, growth pre-hormone, containing a signal peptide which may be released during the transit into the endoplasmic reticulum.

EXAMPLE 3

(A) Example 1 is repeated using plasmid pBR315 in place of plasmid pBR322. All conditions including selection of recombinant clones are as described. The insert is removed and analyzed as described in Example 2 yielding a DNA of about 831 base pairs having the sequence shown in Figure 1.

(B) Example 1 is repeated using plasmic pBR316 in place of plasmid pBR322. All conditions are identical to those described in Example 1. Removal and analysis of the insert as described in Example 2 yields a DNA having the sequence shown in Figure 1.

EXAMPLE 4

For this example, the cDNA coding for bovine pre-growth hormone is prepared as described in Example 1. All restriction site linkers utilized in this example and those that follow are prepared as described by Scheller, R.H. et al., **Science 196,** 177 (1977). The restriction endonucleases used in this Example and those that follow are commercially available from New England Biolabs, Beverly, Massachusetts, and optical conditions according to supplier's recommendations are utilized. Transformation of E. coli $\chi$1776 is performed as described in Example 1.

(A) Hind III linkers having the sequence 5' - CCAAGCTTGG - 3' are ligated to the cDNA prepared in Example 1 by blunt-end ligation using T4 DNA ligase as described by Valenzuela, P., et al., **Nature 280,** 815 (1979). After ligation, the product is digested with Hind III or Hsu I following the procedure described by Ullrich, A. et al., **Science 196,** 1313 (1977). Plasmid pBR322 is cleaved at the Hind III restriction site with either Hind III or Hsu I and treated with alkaline phosphatase as described by Ullrich, A. et al., supra. After ethanol precipitation, the phosphatase-treated, cleaved pBR322 is ligated to the cDNA containing Hind III cohesive termini as described by Ullrich, A., et al., supra. E. coli $\chi$1776 is transformed with the ligation mixture and the transformed colonies are selected for tetracycline sensitivity. The presence of cloned DNA is determined as described in Example 1. A colony containing an insert which hybridized with the nick-translated DNA is obtained. The insert is removed by digestion with Hind III or Hsu I and analyzed as described in Example 2. The insert contains about 830 base pairs and has the sequence shown in Figure 1.

(B) Example 4(A) is repeated in which several vectors are utilized in place of pBR322. In this Example, the plasmids pSC101, pMB9, pBR313, pBR315, and pBR316 are utilized in place of pBR322. All conditions including selection of recombinant clones are as described in (A). For all plasmids, identical results are obtained, i.e., a recombinant plasmid containing an insert having the sequence shown in Figure 1 is obtained in each instance.

(C) Example 4(A) is repeated in which several other restriction site linkers and restriction endonucleases are utilized. In this Example, the linkers for Sal I and Bam HI are used in place of the linkers for Hind III. The sequence of these linkers are 5' - GGTCGACC - 3' and 5' - CCGGATCCGG - 3'. When Sal I linkers are used, the linker treated cDNA and pBR322 are cleaved with restriction endonuclease Sal I and when Bam HI linkers are utilized, the restriction endonuclease is Bam HI. All conditions including selection of recombinant clones are as described in 4(A). For both linkers and restriction endonucleases, identical results are obtained.

(D) Example 4(C) is repeated in which plasmids pSC101, pMB9, pBR313, pBR315 and pBR316 are used in place of pBR322. All conditions are the same as described in Example 4(C) and identical results are obtained, i.e., in each instance an insert having the sequence shown in Figure 1 is obtained.

(E) Example 4(A) is repeated in which an Eco RI restriction linker having the sequence 5' - CCGAATTCGG - 3' is used in place of the Hind III linker and Eco RI is used in place of Hind III (Hsu I). All conditions are identical to those described except that transformed colonies are not selected by tetracycline sensitivity. The presence of cloned DNA is performed as described in Example 1. A recombinant clone containing an insert having the sequence shown in Figure 1 is obtained.

(F) Example 4(E) is repeated in which plasmids pSC101, pMB9, pBR313 and pBR315 are used in place of pBR322. The same conditions as described in Example 4(E) are utilized and identical results are obtained for each plasmid.

(G) Example 4(A) is repeated in which a Pst I restriction linker having the sequence 5' - GCTGCAGC - 3' and Pst I are used in place of the Hind III linker and Hind III (Hsu I), respectively. Identical conditions are employed except that selection of recombinant clones is accomplished as described in Example 1. A recombinant clone having the Figure 1 sequence is obtained.

11

(H) Example 4(G) is repeated in which plasmids pBR315 and pBR316 are used in place of pBR322. The same conditions as described in Example 4(G) are utilized and identical results are obtained.

(I) Examples 1, 3(A), 3(B) and 4(A) - (H) are repeated using E. coli RRI or E. coli HB101 in place of E. coli χ1776. All conditions are as described and identical results are obtained.

## EXAMPLE 5

For this example, the cDNA coding for bovine pre-growth hormone is prepared as described in Example 1. Eco RI restriction site linkers are blunt-end ligated to the cDNA as described by Valenzuela, P., et al., supra, and cleaved with Eco RI as described by Ullrich, A., et al., supra.

(A) Charon 16A DNA prepared as described by Blattner et al., supra, is utilized as the transfer vector. The cohesive ends are annealed by incubation for 60 minutes at 42° C in 0.1M Tris-HCl, pH 8.0 and 10 mm Mg Cl₂. The vector is cleaved at the Eco RI restriction site with Eco RI endonuclease and then treated with alkaline phosphatase as described by Ullrich, A. et al., supra. After ethanol precipitation, the phosphatase-treated vector DNA is added to cDNA containing Eco RI cohesive termini at a molar ratio of 2 moles vector to 1 mole cDNA. The mixture is ligated with T4 DNA ligase as described by Ullrich, A., et al., supra. The ligation mixture is added directly to a suspension of E. coli χ1776 cells prepared for transformation as described in Example 1 and the transformation is performed as described in Example 1. Recombinant phages are recovered and plated on Lac⁻ bacteria on plates containing 5-chloro-4-bromo-3-indolyl-β-D-glactoside (X6), (80 ug/ml) recombinant phages containing the cDNA inserted into the Eco RI site of Charon 16A are selected for the production of colorless plaques. A selected recombinant is isolated and digested with an excess of Eco RI endonuclease and the digest analyzed as described in Example 2. A DNA of about 830 base pairs in length and having the sequence shown in Figure 1 is recovered. Alternatively, the ligation mixture is utilized to form recombinant phages by in vitro packaging as described by Sternberg, N. et al., **Gene 1,** 255 (1977). Recombinant phages can also be screened by utilizing the in situ plaque hybridization technique of Benton, W.D. amd Davis, R.W., **Science 196,** 180 (1977).

(B) Charon 3A or 4A DNA prepared as described by Blattner, et al., supra, is used as the transfer vector in place of the Charon 16A DNA used above. All conditions are identical to those described for Charon 16A DNA. Selection of recombinant phages is performed by (a) plating the phages on Lac⁺ bacteria on plates containing X6 and isolating colorless plaques followed by either hybridization to a suitable probe or restriction endonuclease digestion as described by Blattner, et al., supra. The insert is removed as described above, yielding a DNA of about 830 base pairs in length and having the sequence shown in Figure 1.

(C) λgt WES • λB DNA prepared as described by Tiemier et al., supra, is utilized as the transfer vector in place of the Charon 16A used above. All conditions are identical to those described for Charon 16A. Selection of recombinant phages is performed by the hybridization method of Benton and Davis, supra. The insert is removed as described above, yielding a DNA of about 830 base pairs in length and having the sequence shown in Figure 1.

(D) Examples 5(A) - (C) are repeated in which E. coli RPI, E. coli HB101, E. coli DP50 or E. coli DP50SupF is used in place of E. coli χ1776. All conditions are identical and identical results are obtained.

## EXAMPLE 6

For this example, the cDNA coding for bovine pre-growth hormone is prepared as described in Example 1. Hind III restriction site linkers are added and the cDNA digested with Hind III or Hsu I as described in Example 4(A).

The plasmid pC194 isolated from S. aureus as described by Ehrlich, S.D., supra, is utilized as the transfer vector. pC194 is cleaved at the Hind III site with Hind III or Hsu I and then treated with alkaline phosphatase as described by Ullrich, A. et al., supra. The cDNA having Hind III cohesive termini is ligated to the cleaved pC194 as described by Ullrich, A., et al., supra. Competence-induction of B. subtilis RUB331 is performed as described by Sgaramella, V., et al., **J.Mol.Biol., 105,** 587 (1976) and Borenstein, S. and Ephrati-Elizue, E., **J.Mol.Biol., 45,** 137 (1969). Transformation of B. subtilis RUB331 is performed using the ligation mixture as described by Sgaramella et al. and Borenstein et al. The cell suspension is plated directly on L plates containing 3 μg of chloramphenicol. A selected recombinant is isolated and digested with HindIII and the digest analyzed as described in Example 2. A DNA of about 830 base pairs in length and having the sequence shown in Figure 1 is recovered.

EP 0 047 600 B1

## EXAMPLE 7

Synthesis of cDNA coding for bovine growth hormone.

(A) Plasmid pBR348 is digested with Hae II endonuclease generating a 1600 base pair fragment. One Hae II site is within the cDNA insert coding for growth pre-hormone and the second site is within the pBR322 portion of the plasmid. The digestion yields the following:

```
              +1   +2
      5' -      C TTC .... 3'
      3' - G CGG AAG .... 5'
```

Bovine GH may begin with either the +1 ala or the +2 phe residue at the $NH_2$ terminal end (Dayhoff et al., supra), thus one might attempt to complete the ala codon or one might attempt to delete it. The deletion approach is accomplished by incubating the DNA with dATP and the Klenow fragment of DNA polymerase I, as the first base of the +2 phe codon (on the $3' \rightarrow 5'$ strand) is A. This reaction yields the following:

```
           +1  +2
     5' - C TTC .... 3'
     3' -   AAG .... 5'
```

The DNA is extracted with phenol and precipitated. Excess dATP and the digested bases are removed by chromatography on G-50 Sephadex. The remaining 5' protruding C of the +1 ala codon is then removed with $S_1$ nuclease as described by Shine et al., **Nature 285**, 456 (1980). This digestion yields:

```
          +2
     5' -   TTC .... 3'
     3' -   AAG .... 5'
```

(B) Example 7(A) is repeated in which the deoxynucleotide sequence coding for bovine pre-growth hormone is removed by digestion of the transfer vectors prepared in Examples 3, 4, 5 and 6 by the appropriate restriction enzyme prior to digestion with Hae II. The transfer vectors and restriction enzymes utilized are shown in Table 3.

Table 3

| Restriction Enzyme | Transfer Vector (Example) |
| --- | --- |
| Pst I | 3A, 3B, 4G, 4H |
| Hind III | 4A, 4B, 6 |
| Sal I | 4C, 4D |
| Bam HI | 4C, 4D |
| Eco RI | 4E, 4F, 5A, 5B, 5C |

After digestion with the appropriate restriction enzyme, the bovine pre-growth sequence is isolated by gel electrophoresis and then treated as described in Example 7(A), yielding identical results.

(C) Examples 7(A) and 7(B) are repeated in which T4 DNA polymerase or 3'-5' exonuclease is utilized in place of the Klenow fragment of DNA polymerase I. All other conditions are identical and the identical sequence of the bovine growth hormone fragment is obtained in each instance.

## EXAMPLE 8

13

(A) The bovine growth hormone fragment

$$+1 \quad +2$$
$$5' - \quad C \ TTC \ .... \ 3'$$
$$3' - G \ CGG \ AAG \ .... \ 5'$$

is prepared by Hae II digestion as described in Example 7(A) or 7(B). The completion approach is accomplished as follows. The DNA is incubated with dATP, dGTP, dCTP, dTTP and the Klenow fragment of DNA polymerase I. This reaction yields:

$$+1 \quad +2$$
$$5' - G \ GCC \ TTC \ .... \ 3'$$
$$3' - G \ CGG \ AAG \ .... \ 5'.$$

The DNA is extracted with phenol and precipitated. The sequence is then incubated with the Klenow fragment of DNA polymerase I in the presence of dCTP. The sequence is then digested with S1 nuclease as described by Shine et al., **Nature 285,** 456 (1980), yielding the sequence:

$$5' - GCC \ TTC \ .... \ 3'$$
$$3' - CGG \ AAG \ .... \ 5'.$$

(B) Example 8(A) is repeated in which T4 DNA polymerase or 3'-5' exonuclease is utilized in place of the Klenow fragment in the second incubation step, i.e., in the presence of dCTP. The other conditions are identical and the identical bovine growth hormone sequence is obtained in each instance.

## EXAMPLE 9

The deoxynucleotide sequence coding for bovine growth hormone is inserted into transfer vectors as previously described for the sequence coding for bovine pre-growth hormone.

(A) Examples 1, 3(A), 3(B), 4(A) - (I), 5(A) - (D) and 6 are repeated in which the DNA prepared in Examples 7(A), 7(B) or 7(C) is utilized in place of the DNA coding for bovine pre-growth hormone. All other conditions are identical. The DNA is removed by digestion with the appropriate restriction enzyme, for example as shown in Table 3, and analyzed as described in Example 2. A DNA having the sequence beginning with the Phe codon at position 2 and continuing to the end of the sequence shown in Figure 1 is obtained in each instance.

(B) Examples 1, 3(A), 3(B), 4(A) - (I), 5(A) - (D) and 6 are repeated in which the DNA prepared in Examples 8(A) or 8(B) is utilized in place of the DNA coding for bovine pre-growth hormone. All other conditions are identical. The DNA is removed by digestion with the appropriate restriction enzyme, for example as shown in Table 3, and analyzed as described in Example 2. A DNA having the sequence beginning with the Ala codon at position 1 and continuing to the end of the sequence shown in Figure 1 is obtained in each instance.

## EXAMPLE 10

Expression of bovine growth hormone. Bovine growth hormone can be expressed by any of the methods described above.

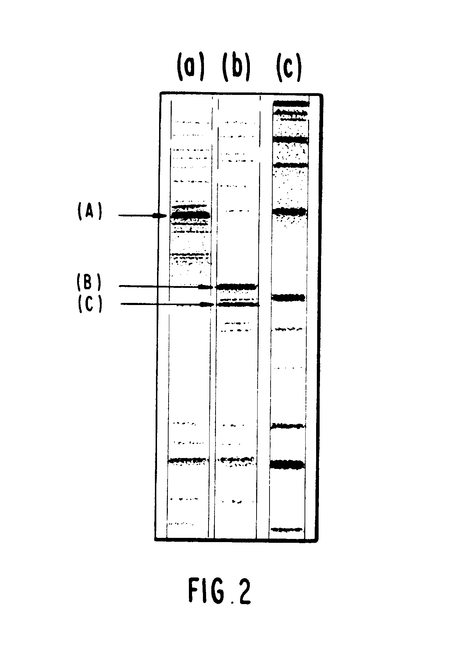

(A) Expression of bovine growth pre-hormone as a fusion protein was demonstrated by performing a radioimmunoassay experiment and performing a minicell experiment. In the radioimmunoassay experiment, E. coli $\chi$1776 containing pBP348 or E. coli containing pBP322 as the control were grown in nutrient broth and collected by centrifugation. The cells were resuspended and lysed and radiolabelled ovine growth hormone and antibody ovine (or bovine) to growth hormone were added The immune complex was precipitated and radioactivity measured. This experiment shows that the fusion protein retains some bovine growth hormone immunoactivity. To further illustrate the production of a fusion

14

protein, a minicell experiment was performed by following the procedure described by Meagher, R.B., et al., **Cell, 10,** 521 (1977). Figure 2 shows the gel electrophoresis bands resulting from E. coli χ1776 minicells. Band (a) was obtained from χ1776 transformed with pBP348. Band (b) was obtained from χ1776 transformed with pBR 322. Band (c) is molecular weight markers. (A) indicates the fusion product of β-lactamase and bovine growth pre-hormone. (B) indicates pre-lactamase and (C) indicates β-lactamase. This experiment shows that pBP348 makes a fusion protein consisting of 183 amino acids of β-lactamase, 217 amino acids of bovine growth pre-hormone and a few linking amino acids coded by the normally untranslated 5' region. The total molecular weight seen, approximately 45,000, agrees with the predicted molecular weight of the hybrid protein.

(B) For the direct expression of bovine growth pre-hormone the insert DNA is first separated from pBP348 by partial Pst I endonuclease digestion and purified by preparative gel electrophoresis. A 15 μg sample of purified insert DNA is then modified by suspending the DNA in water to which is added a concentrated solution of salts such that the final composition comprises 70mM Tris, pH 8.8, 70mM $MgCl_2$, 10mM dithiothreitol and 13.75 units of T4 DNA polymerase in a total volume of 250 μl. The reaction mixture is incubated at 37°C for several minutes and then dATP is added to a concentration of 50 mM to terminate endonucleolytic digestion at the next adenine residue. After 30 seconds of additional incubation, the enzyme is inactivated by heat treatment at 65°C for five minutes. This process is repeated twice more, once in which dCTP is used in place of dATP and finally in which dTTP is again used. The treated DNA is recovered by ethanol precipitation. Digestion with S1 nuclease to provide blunt ends is carried out as described by Ullrich, A., et al., supra. This procedure is designed to produce a DNA molecule terminated at the start codon at the position number -26. Such molecules will be translated when inserted in an expression vector having an insertion site about 3-11 nucleotides from the ribosome binding site sequence of an expression unit.

A vector for direct expression is constructed by modification of the plasmid ptrpE30 by the removal of 23-29 nucleotides using T4 DNA polymerase and S1 nuclease as described above.

The modified cDNA and the modified expression vector are provided with a specific linker having the sequence 5'-CCGGATCCGG-3' on one strand and its complementary sequence on the other by blunt-end ligation using DNA ligase as described by Valenzuela, supra. The linkers provide restriction sites sensitive to Bam HI endonuclease which are employed to facilitate insertion. Insertion is accomplished by following the procedure of Ullrich, A., et al., supra. Host bacteria E. coli HB101, RRI or χ1776 are transformed by the recombinant vectors bearing the inserted modified growth pre-hormone coding region and transformants are selected for resistance to ampicillin. A single transformant designated ptrpE30/bGH is selected for further analysis.

Bacterial cells transformed by ptrpE30/bGH are grown in a standard minimal medium (M9) supplemented with Leu, Pro, vitamin B1 and ampicillin at 37°C. In early log phase, the trp operon is induced by the addition of β-indolylacrylic acid (30 μg/ml medium). Control cultures are left uninduced. After three more hours of growth, 1.5 ml of cells are radioactively labelled by the addition of 20 μCi of $^{35}$S-L-Met and incubated for 10 minutes. The cells are collected by centrifugation, washed and resuspended in 250 μl of buffer containing 10% (v/v) glycol, 5% (v/v) β-mercaptoethanol and 2.3% (w/v) SDS in 0.0625M Tris, pH 6.8. The suspension is boiled for five minutes, then applied to a 10% (w/v) SDS-polyacrylamide gel and fractionated by electrophoresis. The protein bands are visualized by autoradiography. The results show the existence of a new protein band of about 24,000 daltons not observed in the uninduced or non-transformed cultures.

The bovine growth pre-hormone is purified by conventional techniques including, for example, gel filtration, ion exchange chromatography, affinity chromatography and differential solubility techniques. Growth pre-hormone is converted to growth hormone by following the procedure described by Jackson, et al., supra.

(C) A specific linker having the sequence 5' - CCGGATCCGGATG - 3' on one strand and its complementary sequence on the other is blunt-end ligated to the DNA coding for bovine growth hormone as prepared in Examples 7(A) - (C) and 8(A) - (B). This modified DNA is then inserted in the modified plasmid ptrpE30 as described in Example 10(B). Host bacteria is transformed and cultured and the resulting bovine growth hormone is purified as described in Example 10(B).

While the invention has been described in connection with specific embodiments thereof, it will be understood that it is capable of further modifications. This application is intended to cover any variations, uses or adaptations of the invention following, in general, the principles of the invention and including such departures from the present disclosure as come within known and customary practice within the art to which the invention pertains.

**Claims**

1. A DNA transfer vector comprising a deoxynucleotide sequence comprising a plus strand having the sequence:

$$5' - ATG \ ATG \ GCT \ GCA \ GGC \ CCC \ CGG \ ACC \ TCC \ CTG$$

CTC CTG GCT TTC GCC CTG CTC TGC CTG CCC TGG ACT CAG GTG

GTG GGC GCC TTC CCA GCC ATG TCC TTG TCC GGC CTG TTT GCC

AAC GCT GTG CTC CGG GCT CAG CAC CTG CAC CAG CTG GCT GCT

GAC ACC TTC AAA GAG TTT GAG CGT ACC TAC ATC CCG GAG GGA

CAG AGA TAC TCC ATC CAG AAC ACC CAG GTT GCC TTC TGC TTC

TCC GAA ACC ATC CCG GCC CCC ACG GGC AAG AAT GAG GCC CAG

CAG AAA TCA GAC TTG GAG CTG CTT CGC ATC TCA CTG CTC CTC

ATC CAG TCG TGG CTT GGG CCC CTG CAG TTT CTC AGC AGA GTC

TTC ACC AAC AGC TTG GTG TTT GGC ACC TCG GAC CGT GTC TAT

GAG AAG CTG AAG GAC CTG GAG GAA GGC ATC TTG GCC CTG ATG

CGG GAG CTG ·GAA GAT GGC ACC CCC CGG GCT GGG CAG ATC CTC

AAG CAG ACC TAT GAC AAA TTT GAC ACA AAC ATG CGC AGT GAC

GAC GCG CTG CTC AAG AAC TAC GGT CTG CTC TCC TGC TTC CGG

AAG GAC CTG CAT AAG ACG GAG ACG TAC CTG AGG GTC ATG AAG

TGC CGC CGC TTC GGG GAG GCC AGC TGT GCC TTC TAG- 3'

wherein

A    is deoxyadenyl,
G    is deoxyguanyl,
C    is deoxycytosyl and
T    is thymidyl,

or a modification thereof, said deoxynucleotide sequence coding for bovine pre-growth hormone.

2. The DNA transfer vector comprising a deoxynucleotide sequence comprising a plus strand having the sequence:

```
                5' - GCC TTC CCA GCC ATG TCC TTG TCC GGC CTG
     TTT GCC AAC GCT GTG CTC CGG GCT CAG CAC CTG CAC CAG CTG
     GCT GCT GAC ACC TTC AAA GAG TTT GAG CCT ACC TAC ATC CCG
     GAG GGA CAG AGA TAC TCC ATC CAG AAC ACC CAG GTT GCC TTC
     TGC TTC TCC GAA ACC ATC CCG GCC CCC ACG GGC AAG AAT GAG
     GCC CAG CAG AAA TCA GAC TTG GAG CTG CTT CGC ATC TCA CTG
     CTC CTC ATC CAG TCG TGG CTT GGG CCC CTG CAG TTT CTC AGC
     AGA GTC TTC ACC AAC AGC TTG GTG TTT GGC ACC TCG GAC CGT
     GTC TAT GAG AAG CTG AAG GAC CTG GAG GAA GGC ATC TTG GCC
     CTG ATG CGG GAG CTG GAA GAT GGC ACC CCC CGG GCT GGG CAG
     ATC CTC AAG CAG ACC TAT GAC AAA TTT GAC ACA AAC ATG CGC
     AGT GAC GAC GCG CTG CTC AAG AAC TAC GGT CTG CTC TCC TGC
     TTC CGG AAG GAC CTG CAT AAG ACG GAG ACG TAC CTG AGG GTC
     ATG AAG TGC CGC CGC TTC GGG GAG GCC AGC TGT GCC TTC TAG
     - 3'
```

wherein

A     is deoxyadenyl,
G     is deoxyguanyl,
C     is deoxycytosyl and
T     is thymidyl,

or a modification thereof, said deoxynucleotide sequence coding for bovine growth hormone.

**3.** The DNA transfer vector of claim 2 modified in that said deoxynucleotide sequence comprises a plus strand having the sequence:

```
                5' - TTC CCA GCC ATG TCC TTG TCC GGC CTG TTT
     GCC AAC GCT GTG CTC CGG GCT CAG CAC CTG CAC CAG CTG GCT
     GCT GAC ACC TTC AAA GAG TTT GAG CGT ACC TAC ATC CCG GAG
     GGA CAG AGA TAC TCC ATC CAG AAC ACC CAG GTT GCC TTC TGC
     TTC TCC GAA ACC ATC CCG GCC CCC ACG GGC AAG AAT GAG GCC
     CAG CAG AAA TCA GAC TTG GAG CTG CTT CGC ATC TCA CTG CTC
     CTC ATC CAG TCG TGG CTT GGG CCC CTG CAG TTT CTC AGC AGA
     GTC TTC ACC AAC AGC TTG GTG TTT GGC ACC TCG GAC CGT GTC
     TAT GAG AAG CTG AAG GAC CTG GAG GAA GGC ATC TTG GCC CTG
     ATG CGG GAG CTG GAA GAT GGC ACC CCC CGG GCT GGG CAG ATC
```

```
CTC AAG CAG ACC TAT GAC AAA TTT GAC ACA AAC ATG CGC AGT

GAC GAC GCG CTG CTC AAG AAC TAC GGT CTG CTC TCC TGC TTC

CGG AAG GAC CTG CAT AAG ACG GAG ACG PAC CTG AGG GTC ATG

AAG TGC CGC CGC TTC GGG GAG GCC AGC TGT GCC TTC TAG - 3'
```

wherein

A    is deoxyadenyl,
G    is deoxyguanyl,
C    is deoxycytosyl and
T    is thymidyl,

or a modification thereof.

4.    A bacterium transformed by the transfer vector of any of claims 1 to 3.

5.    The bacterium of claim 4 wherein the bacterium is *Escherichia coli.*

6.    The bacterium of claim 5 wherein the bacterium is selected from the group comprising *Escherichia coli* χ 1776, HB101 or RRI.

7.    A process for making a DNA transfer vector for use in maintaining and replicating a deoxynucleotide sequence coding for bovine pre-growth hormone, comprising reacting a deoxynucleotide sequence coding for bovine pre-growth hormone with a DNA molecule prepared by cleaving a transfer vector with a restriction enzyme, said deoxynucleotide sequence comprising a plus strand having the sequence:

```
5' - ATG ATG GCT GCA GGC CCC CGG ACC TCC CTG

CTC CTG GCT TTC GCC CTG CTC TGC CTG CCC TGG ACT CAG GTC

GTG GGC GCC TTC CCA GCC ATG TCC TTG TCC GGC CTG TTT GCC

AAC GCT GTG CTC CGG GCT CAG CAC CTG CAC CAG CTG GCT GCT

GAC ACC TTC AAA GAG TTT GAG CGT ACC TAC ATC CCG GAG GGA

CAG AGA TAC TCC ATC CAG AAC ACC CAG GTT GCC TTC TGC TTC

TCC GAA ACC ATC CCG GCC CCC ACG GGC AAG AAT GAG GCC CAG

CAG AAA TCA GAC TTG GAG CTG CTT CGC ATC TCA CTG CTC CTC

ATC CAG TCG TGG CTT GGG CCC CTG CAG TTT CTC AGC AGA GTC

TTC ACC AAC AGC TTG GTG TTT GGC ACC TCG GAC CGT GTC TAT

GAG AAG CTG AAG GAC CTG GAG GAA GGC ATC TTG GCC CTG ATG

CGG GAG CTG GAA GAT GGC ACC CCC CGG GCT GGG CAG ATC CTC

AAG CAG ACC TAT GAC AAA TTT GAC ACA AAC ATG CGC AGT GAC

GAC GCG CTG CTC AAG AAC TAC GGT CTG CTC TCC TGC TTC CGG

AAG GAC CTG CAT AAG ACG GAG ACG TAC CTG AGG GTC ATG AAG

TGC CGC CGC TTC GGG GAG GCC AGC TGT GCC TTC TAG - 3'
```

wherein

A    is deoxyadenyl,
G    is deoxyguanyl,
C    is deoxycytosyl and

T    is thymidyl;
or a modification thereof, said deoxynucleotide sequence codi. .. for bovine pre-growth hormone

8. A process for preparing a deoxynucleotide sequence for use in synthesizing bovine growth hormone or a substantial part thereof characterised by cleaving a cDNA coding for bovine pre-growth hormone, maintained and replicated by means of a vector prepared according to the process of claim 7, with the restriction enzyme Hae II and incubating the cleaved product with an enzyme selected from the group comprising the Klenow fragment of DNA polymerase I, T4 DNA polymerase or 3'-5' exonuclease.

9. The process of claim 8 characterised in that the incubation with said Klenow fragment is conducted in the presence of dATP and the resulting product is digested with S1 nuclease whereby a deoxynucleotide sequence coding for amino acids 2-191 of bovine growth hormone is obtained.

10. The process of claim 9 characterised in that the deoxynucleotide sequence coding for bovine growth hormone comprises a plus strand having the sequence:

5' - TTC CCA GCC ATG TCC TTG TCC GGC CTG TTT

GCC AAC GCT GTG CTC CGG GCT CAG CAC CTG CAC CAG CTG GCT

GCT GAC ACC TTC AAA GAG TTT GAG CGT ACC TAC ATC CCG GAG

GGA CAG AGA TAC TCC ATC CAG AAC ACC CAG GTT GCC TTC TGC

TTC TCC GAA ACC ATC CCG GCC CCC ACG GGC AAG AAT GAG GCC

CAG CAG AAA TCA GAC TTG GAG CTG CTT CGC ATC TCA CTG CTC

CTC ATC CAG TCG TGG CTT GGG CCC CTG CAG TTT CTC AGC AGA

GTC TTC ACC AAC AGC TTG GTG TTT GGC ACC TCG GAC CGT GTC

TAT GAG AAG CTG AAG GAC CTG GAG GAA GGC ATC TTG GCC CTG

ATG CGG GAG CTG GAA GAT GGC ACC CCC CGG GCT GGG CAG ATC

CTC AAG CAG ACC TAT GAC AAA TTT GAC ACA AAC ATG CGC AGT

GAC GAC GCG CTG CTC AAG AAC TAC GGT CTG CTC TCC TGC TTC

CGG AAG GAC CTG CAT AAG ACG GAG ACG TAC CTG AGG GTC ATG

AAG TGC CGC CGC TTC GGG GAG GCC AGC TGT GCC TTC TAG - 3'

wherein
A    is deoxyadenyl,
G    is deoxyguanyl,
C    is deoxycytosyl and
T    is thymidyl;
or a modification thereof, said deoxynucleotide sequence coding for bovine growth hormone.

11. The process of claim 8 characterised in that the incubation with said Klenow fragment is conducted in the presence of dATP, dGTP, dCTP and dTTP, the resulting product is incubated with an enzyme selected from the group comprising said Klenow fragment, T4 DNA polymerase or 3'-5' exonuclease in the presence of dCTP and digesting the resulting product with S1 nuclease whereby a deoxynucleotide sequence coding for amino acids 1-191 of bovine growth hormone is obtained.

12. The process of claim 11 characterised in that the deoxynucleotide sequence coding for bovine growth hormone comprises a plus strand having the sequence:

19

```
5' - GCC TTC CCA GCC ATG TCC TTG TCC GGC CTG
TTT GCC AAC GCT GTG CTC CGG GCT CAG CAC CTG CAC CAG CTG
GCT GCT GAC ACC TTC AAA GAG TTT GAG CGT ACC TAC ATC CCG
GAG GGA CAG AGA TAC TCC ATC CAG AAC ACC CAG GTT GCC TTC
TGC TTC TCC GAA ACC ATC CCG GCC CCC ACG GGC AAG AAT GAG
GCC CAG CAG AAA TCA GAC TTG GAG CTG CTT CGC ATC TCA CTG
CTC CTC ATC CAG TCG TGG CTT GGG CCC CTG CAG TTT CTC AGC
AGA GTC TTC ACC AAC AGC TTG GTG TTT GGC ACC TCG GAC CGT
GTC TAT GAG AAG CTG AAG GAC CTG GAG GAA GGC ATC TTG GCC
CTG ATG CGG GAG CTG GAA GAT GGC ACC CCC CGG GCT GGG CAG
ATC CTC AAG CAG ACC TAT GAC AAA TTT GAC ACA AAC ATG CGC
AGT GAC GAC GCG CTG CTC AAG AAC TAC GGT CTG CTC TCC TGC
TTC CGG AAG GAC CTG CAT AAG ACG GAG ACG TAC CTG AGG GTC
ATG AAG TGC CGC CGC TTC GGG GAG GCC AGC TGT GCC TTC TAG
- 3'
```

wherein

A    is deoxyadenyl,
G    is deoxyguanyl,
C    is deoxycytosyl and
T    is thymidyl;

or a modification thereof.

**13.** A process according to claim 7 characterised in that the DNA molecule prepared by cleaving a transfer vector with a restriction enzyme is pBR322 and the restriction enzyme is Pst I.

**14.** A process for making a DNA transfer vector for use in maintaining and replicating a deoxynucleotide sequence coding for bovine growth hormone characterised by reacting a deoxynucleotide sequence coding for bovine growth hormone, made according to any one of claims 8 to 12, with a DNA molecule prepared by cleaving a transfer vector with a restriction enzyme.

**15.** A process for making an expression transfer vector in accordance with claim 7 or claim 14 for use in expressing a deoxynucleotide sequence coding for bovine pre-growth hormone or bovine growth hormone characterised in that the DNA molecule prepared by cleaving a transfer vector with a restriction enzyme is cleaved at a point within an expressible control region.

**16.** A process for making a fusion protein comprising the amino acid sequence of bovine pre-growth hormone or the amino acid sequence of bovine growth hormone as its C-terminal end and a portion of procaryotic protein as its N-terminal end characterized by incubating a microorganism transformed by an expression transfer vector comprising a deoxynucleotide sequence coding for said bovine pre-growth hormone or for said bovine growth hormone prepared in accordance with claim 15.

**17.** A process for synthesizing bovine growth hormone characterised by incubating a microorganism transformed by an expression transfer vector comprising a deoxynucleotide sequence coding for said bovine growth hormone prepared in accordance with any of claims 8 to 12 under conditions suitable for expression of said sequence coding for bovine growth hormone and purifying bovine growth hormone from the lysate or culture medium of said microorganism.

**18.** A process for synthesizing bovine growth hormone or bovine pre-growth hormone, characterised by incubating a microorganism transformed by an expression transfer vector comprising a DNA sequence as recited in any of claims 1 to 3, under conditions suitable for expression of said sequence coding for bovine growth hormone or bovine pre-growth hormone and purifying bovine growth hormone or bovine pre-growth hormone from the lysate or culture medium of said microorganism.

**Revendications**

**1.** Un vecteur de transfert de DNA comprenant une séquence de déoxynucléotides comprenant un brin plus ayant la séquence:

```
       5' - ATG ATG GCT GCA GGC CCC CGG ACC TCC CTG
   CTC CTG GCT TTC GCC CTG CTC TGC CTG CCC TGG ACT CAG GTG
   GTG GGC GCC TTC CCA GCC ATG TCC TTG TCC GGC CTG TTT GCC
   AAC GCT GTG CTC CGG GCT CAG CAC CTG CAC CAG CTG GCT GCT
   GAC ACC TTC AAA GAG TTT GAG CGT ACC TAC ATC CCG GAG GGA
   CAG AGA TAC TCC ATC CAG AAC ACC CAG GTT GCC TTC TGC TTC
   TCC GAA ACC ATC CCG GCC CCC ACG GGC AAG AAT GAG GCC CAG
   CAG AAA TCA GAC TTG GAG CTG CTT CGC ATC TCA CTG CTC CTC
   ATC CAG TCG TGG CTT GGG CCC CTG CAG TTT CTC AGC AGA GTC
   TTC ACC AAC AGC TTG GTG TTT GGC ACC TCG GAC CGT GTC TAT
   GAG AAG CTG AAG GAC CTG GAG GAA GGC ATC TTG GCC CTG ATG
   CGG GAG CTG GAA GAT GGC ACC CCC CGG GCT GGG CAG ATC CTC
   AAG CAG ACC TAT GAC AAA TTT GAC ACA AAC ATG CGC AGT GAC
   GAC GCG CTG CTC AAG AAC TAC GGT CTG CTC TCC TGC TTC CGG
   AAG GAC CTG CAT AAG ACG GAG ACG TAC CTG AGG GTC ATG AAG
   TGC CGC CGC TTC GGG GAG GCC AGC TGT GCC TTC TAG- 3'
```

dans laquelle

A    est déoxyadényl,
G    est déoxyguanyl,
C    est déoxycytosyl et
T    est thymidyl,

ou une modification de celle-ci, ladite séquence de déoxynucléotide codant pour l'hormone de pré-croissance bovine.

**2.** Un vecteur de transfert de DNA comprenant une séquence de déoxynucléotides comprenant un brin plus ayant la séquence:

21

5' – GCC TTC CCA GCC ATG TCC TTG TCC GGC CTG
TTT GCC AAC GCT GTG CTC CGG GCT CAG CAC CTG CAC CAG CTG
GCT GCT GAC ACC TTC AAA GAG TTT GAG CCT ACC TAC ATC CCG
GAG GGA CAG AGA TAC TCC ATC CAG AAC ACC CAG GTT GCC TTC
TGC TTC TCC GAA ACC ATC CCG GCC CCC ACG GGC AAG AAT GAG
GCC CAG CAG AAA TCA GAC TTG GAG CTG CTT CGC ATC TCA CTG
CTC CTC ATC CAG TCG TGG CTT GGG CCC CTG CAG TTT CTC AGC
AGA GTC TTC ACC AAC AGC TTG GTG TTT GGC ACC TCG GAC CGT
GTC TAT GAG AAG CTG AAG GAC CTG GAG GAA GGC ATC TTG GCC
CTG ATG CGG GAG CTG GAA GAT GGC ACC CCC CGG GCT GGG CAG
ATC CTC AAG CAG ACC TAT GAC AAA TTT GAC ACA AAC ATG CGC
AGT GAC GAC GCG CTG CTC AAG AAC TAC GGT CTG CTC TCC TGC
TTC CGG AAG GAC CTG CAT AAG ACG GAG ACG TAC CTG AGG GTC
ATG AAG TGC CGC CGC TTC GGG GAG GCC AGC TGT GCC TTC TAG
– 3'

dans laquelle
A est déoxyadényl,
G est déoxyguanyl,
C est déoxycytosyl et
T est thymidyl,
ou une modification de celle-ci, ladite séquence de déoxynucléotides codant pour l'hormone de croissance bovine.

3. Le vecteur de transfert de DNA de la revendication 2 modifié pour que la séquence de déoxynucléotides comprenne un brin plus ayant la séquence:

```
        5' - TTC CCA GCC ATG TCC TTG TCC GGC CTG TTT

GCC AAC GCT GTG CTC CGG GCT CAG CAC CTG CAC CAG CTG GCT

GCT GAC ACC TTC AAA GAG TTT GAG CGT ACC TAC ATC CCG GAG

GGA CAG AGA TAC TCC ATC CAG AAC ACC CAG GTT GCC TTC TGC

TTC TCC GAA ACC ATC CCG GCC CCC ACG GGC AAG AAT GAG GCC

CAG CAG AAA TCA GAC TTG GAG CTG CTT CGC ATC TCA CTG CTC

CTC ATC CAG TCG TGG CTT GGG CCC CTG CAG TTT CTC AGC AGA

GTC TTC ACC AAC AGC TTG GTG TTT GGC ACC TCG GAC CGT GTC

TAT GAG AAG CTG AAG GAC CTG GAG GAA GGC ATC TTG GCC CTG

ATG CGG GAG CTG GAA GAT GGC ACC CCC CGG GCT GGG CAG ATC

CTC AAG CAG ACC TAT GAC AAA TTT GAC ACA AAC ATG CGC AGT

GAC GAC GCG CTG CTC AAG AAC TAC GGT CTG CTC TCC TGC TTC

CGG AAG GAC CTG CAT AAG ACG GAG ACG PAC CTG AGG GTC ATG

AAG TGC CGC CGC TTC GGG GAG GCC AGC TGT GCC TTC TAG - 3'
```

dans laquelle

A     est déoxyadényl,

G     est déoxyguanyl,

C     est déoxycytosyl et

T     est thymidyl,

ou une modification de celle-ci.

**4.** Une bactérie transformée par le vecteur de transfert de l'une quelconque des revendications 1 à 3.

**5.** La bactérie de la revendication 4 dans laquelle la bactérie est Escherichia coli.

**6.** La bactérie de la revendication 5 dans laquelle la bactérie est choisie dans le groupe constitué par Escherichia coli x 1776, HB101 ou RRI.

**7.** Un procédé pour préparer un vecteur de transfert de DNA destiné à être utilisé pour maintenir et répliquer une séquence de déoxynucléotides codant pour l'hormone de pré-croissance bovine, consistant à faire réagir une séquence de désoxynucléotides codant pour l'hormone de pré-croissance bovine avec une molécule de DNA préparée en coupant un vecteur de transfert au moyen d'une enzyme de restriction, ladite séquence de déoxynucléotides comprenant un brin plus ayant la séquence:

```
5' - ATG ATG GCT GCA GGC CCC CGG ACC TCC CTG
CTC CTG GCT TTC GCC CTG CTC TGC CTG CCC TGG ACT CAG GTC
GTG GGC GCC TTC CCA GCC ATG TCC TTG TCC GGC CTG TTT GCC
AAC GCT GTG CTC CGG GCT CAG CAC CTG CAC CAG CTG GCT GCT
GAC ACC TTC AAA GAG TTT GAG CGT ACC TAC ATC CCG GAG GGA
CAG AGA TAC TCC ATC CAG AAC ACC CAG GTT GCC TTC TGC TTC
TCC GAA ACC ATC CCG GCC CCC ACG GGC AAG AAT GAG GCC CAG
CAG AAA TCA GAC TTG GAG CTG CTT CGC ATC TCA CTG CTC CTC
ATC CAG TCG TGG CTT GGG CCC CTG CAG TTT CTC AGC AGA GTC
TTC ACC AAC AGC TTG GTG TTT GGC ACC TCG GAC CGT GTC TAT
GAG AAG CTG AAG GAC CTG GAG GAA GGC ATC TTG GCC CTG ATG
CGG GAG CTG GAA GAT GGC ACC CCC CGG GCT GGG CAG ATC CTC
AAG CAG ACC TAT GAC AAA TTT GAC ACA AAC ATG CGC AGT GAC
GAC GCG CTG CTC AAG AAC TAC GGT CTG CTC TCC TGC TTC CGG
AAG GAC CTG CAT AAG ACG GAG ACG TAC CTG AGG GTC ATG AAG
TGC CGC CGC TTC GGG GAG GCC AGC TGT GCC TTC TAG - 3'
```

dans laquelle

    A     est déoxyadényl,
    G     est déoxyguanyl,
    C     est déoxycytosyl et
    T     est thymidyl;

ou une modification de celle-ci, ladite séquence de déoxynucléotides codant pour l'hormone de pré-croissance bovine.

8. Un procédé pour préparer une séquence de déoxynucléotides destinée à être utilisée pour synthétiser l'hormone de croissance bovine ou une partie substantielle de celle-ci, caractérisé par le fait qu'on coupe un cDNA codant pour l'hormone de pré-croissance bovine, maintenu et répliqué au moyen d'un vecteur préparé selon le procédé de la revendication 7, avec l'enzyme de restriction Hae II, et qu'on incube le produit coupé avec une enzyme choisie dans le groupe comprenant le fragment de Klenow de la DNA polymérase I, de la T4 DNA polymérase ou de la 3'-5' exonucléase.

9. Le procédé de la revendication 8, caractérisé en ce que l'incubation avec ledit fragment de Klenow est réalisée en présence de dATP et que le produit résultant est digéré avec la S1 nucléase, grâce à quoi on obtient une séquence de déoxynucléotides codant pour les acides aminés 2-191 de l'hormone de croissance bovine.

10. Le procédé de la revendication 9, caractérisé en ce que la séquence de déoxynucléotides codant pour l'hormone de croissance bovine comprend un brin plus ayant la séquence:

```
        5' - TTC CCA GCC ATG TCC TTG TCC GGC CTG TTT

GCC AAC GCT GTG CTC CGG GCT CAG CAC CTG CAC CAG CTG GCT

GCT GAC ACC TTC AAA GAG TTT GAG CGT ACC TAC ATC CCG GAG

GGA CAG AGA TAC TCC ATC CAG AAC ACC CAG GTT GCC TTC TGC

TTC TCC GAA ACC ATC CCG GCC CCC ACG GGC AAG AAT GAG GCC

CAG CAG AAA TCA GAC TTG GAG CTG CTT CGC ATC TCA CTG CTC

CTC ATC CAG TCG TGG CTT GGG CCC CTG CAG TTT CTC AGC AGA

GTC TTC ACC AAC AGC TTG GTG TTT GGC ACC TCG GAC CGT GTC

TAT GAG AAG CTG AAG GAC CTG GAG GAA GGC ATC TTG GCC CTG

ATG CGG GAG CTG GAA GAT GGC ACC CCC CGG GCT GGG CAG ATC

CTC AAG CAG ACC TAT GAC AAA TTT GAC ACA AAC ATG CGC AGT

GAC GAC GCG CTG CTC AAG AAC TAC GGT CTG CTC TCC TGC TTC

CGG AAG GAC CTG CAT AAG ACG GAG ACG TAC CTG AGG GTC ATG

AAG TGC CGC CGC TTC GGG GAG GCC AGC TGT GCC TTC TAG - 3'
```

dans laquelle

A    est déoxyadényl,
G    est déoxyguanyl,
C    est déoxycytosyl et
T    est thymidyl;

ou une modification de celle-ci, ladite séquence de déoxynucléotides codant pour l'hormone de croissance bovine.

**11.** Le procédé de la revendication 8, caractérisé en ce que l'incubation au moyen dudit fragment de Klenow est réalisée en présence de dATP, dGTP, dCTP et dTTP, le produit résultant est incubé au moyen d'une enzyme choisie dans le groupe comprenant ledit fragment de Klenow, la T4 DNA polymérase ou la 3'-5' exonucléase en présence de dCTP et le produit résultant est digéré au moyen de S1 nucléase, grâce à quoi on obtient une séquence de déoxynucléotides codant pour les acides aminés 1-191 de l'hormone de croissance bovine.

**12.** Le procédé de la revendication 11, caractérisé en ce que la séquence de déoxynucléotides codant pour l'hormone de croissance bovine comprend un brin plus ayant la séquence:

```
5' - GCC TTC CCA GCC ATG TCC TTG TCC GGC CTG
TTT GCC AAC GCT GTG CTC CGG GCT CAG CAC CTG CAC CAG CTG
GCT GCT GAC ACC TTC AAA GAG TTT GAG CGT ACC TAC ATC CCG
GAG GGA CAG AGA TAC TCC ATC CAG AAC ACC CAG GTT GCC TTC
TGC TTC TCC GAA ACC ATC CCG GCC CCC ACG GGC AAG AAT GAG
GCC CAG CAG AAA TCA GAC TTG GAG CTG CTT CGC ATC TCA CTG
CTC CTC ATC CAG TCG TGG CTT GGG CCC CTG CAG TTT CTC AGC
AGA GTC TTC ACC AAC AGC TTG GTG TTT GGC ACC TCG GAC CGT
GTC TAT GAG AAG CTG AAG GAC CTG GAG GAA GGC ATC TTG GCC
CTG ATG CGG GAG CTG GAA GAT GGC ACC CCC CGG GCT GGG CAG
ATC CTC AAG CAG ACC TAT GAC AAA TTT GAC ACA AAC ATG CGC
AGT GAC GAC GCG CTG CTC AAG AAC TAC GGT CTG CTC TCC TGC
TTC CGG AAG GAC CTG CAT AAG ACG GAG ACG TAC CTG AGG GTC
ATG AAG TGC CGC CGC TTC GGG GAG GCC AGC TGT GCC TTC TAG
- 3'
```

dans laquelle
    A    est déoxyadényl,
    G    est déoxyguanyl,
    C    est déoxycytosyl et
    T    est thymidyl;
ou une modification de celle-ci.

**13.** Un procédé selon la revendication 7, caractérisé en ce que la molécule de DNA préparée en coupant un vecteur de transfert au moyen d'une enzyme de restriction est pBR322 et l'enzyme de restriction est Pst I.

**14.** Un procédé pour préparer un vecteur de transfert de DNA, destiné à être utilisé pour maintenir et répliquer une séquence de déoxynucléotides codant pour l'hormone de croissance bovine, caractérisé en ce qu'on fait réagir une séquence de déoxynucléotides codant pour l'hormone de croissance bovine, préparée selon l'une des revendications 8 à 12, avec une molécule de DNA préparée en coupant un vecteur de transfert au moyen d'une enzyme de restriction.

**15.** Un procédé pour préparer un vecteur de transfert d'expression en conformité avec la revendication 7 ou la revendication 14, destiné à être utilisé pour exprimer une séquence de déoxynucléotides codant pour l'hormone de pré-croissance bovine ou l'hormone de croissance bovine, caractérisé en ce que la molécule de DNA préparée en coupant un vecteur de transfert au moyen d'une enzyme de restriction est coupé en un point compris à l'intérieur d'une région de contrôle expressible.

**16.** Un procédé pour préparer une protéine de fusion comprenant la séquence d'acides aminés de l'hormone de pré-croissance bovine ou la séquence d'acides aminés de l'hormone de croissance bovine en tant que son extrémité terminale C et une portion de la protéine procaryote en tant que son extrémité N, caractérisé en ce qu'on incube un micro-organisme transformé par un vecteur de transfert d'expression comprenant une séquence de déoxynucléotides codant pour ladite hormone de pré-croissance bovine ou pour ladite hormone de croissance bovine préparée en conformité avec la revendication 15.

**17.** Un procédé pour synthétiser une hormone de croissance bovine caractérisé en ce qu'on incube un micro-organisme transformé par un vecteur de transfert d'expression comprenant une séquence de déoxynucléotides codant pour ladite hormone de croissance bovine préparée en conformité avec l'une quelconque des revendications 8 à 12 dans des conditions convenables pour l'expression de ladite séquence codant pour l'hormone de croissance bovine et en ce qu'on purifie l'hormone de croissance bovine à partir du lysat ou du milieu de culture dudit micro-organisme.

18. Un procédé pour synthétiser une hormone de croissance bovine ou une hormone de pré-croissance bovine, caractérisé en ce qu'on incube un micro-organisme transformé par un vecteur de transfert d'expression comprenant une séquence de DNA telle que précisée dans l'une quelconque des revendications 1 à 3, dans des conditions convenant pour l'expression de ladite séquence codant pour l'hormone de croissance bovine ou l'hormone de pré-croissance bovine, et qu'on purifie l'hormone de croissance bovine ou l'hormone de pré-croissance bovine à partir du lysat ou du milieu de culture dudit micro-organisme.

**Patentansprüche**

1. DNA-Transfervektor, der eine Desoxynucleotidsequenz umfaßt, die aus einem Plus-Strang mit der Sequenz besteht:

```
5' - ATG ATG GCT GCA GGC CCC CGG ACC TCC CTG
CTC CTG GCT TTC GCC CTG CTC TGC CTG CCC TGG ACT CAG GTG
GTG GGC GCC TTC CCA GCC ATG TCC TTG TCC GGC CTG TTT GCC
AAC GCT GTG CTC CGG GCT CAG CAC CTG CAC CAG CTG GCT GCT
GAC ACC TTC AAA GAG TTT GAG CGT ACC TAC ATC CCG GAG GGA
CAG AGA TAC TCC ATC CAG AAC ACC CAG GTT GCC TTC TGC TTC
TCC GAA ACC ATC CCG GCC CCC ACG GGC AAG AAT GAG GCC CAG
CAG AAA TCA GAC TTG GAG CTG CTT CGC ATC TCA CTG CTC CTC
ATC CAG TCG TGG CTT GGG CCC CTG CAG TTT CTC AGC AGA GTC
TTC ACC AAC AGC TTG GTG TTT GGC ACC TCG GAC CGT GTC TAT
GAG AAG CTG AAG GAC CTG GAG GAA GGC ATC TTG GCC CTG ATG
CGG GAG CTG GAA GAT GGC ACC CCC CGG GCT GGG CAG ATC CTC
AAG CAG ACC TAT GAC AAA TTT GAC ACA AAC ATG CGC AGT GAC
GAC GCG CTG CTC AAG AAC TAC GGT CTG CTC TCC TGC TTC CGG
AAG GAC CTG CAT AAG ACG GAG ACG TAC CTG AGG GTC ATG AAG
TGC CGC CGC TTC GGG GAG GCC AGC TGT GCC TTC TAG- 3'
```

worin
A    Desoxyadenyl,
G    Desoxyguanyl,
C    Desoxycytosyl und
T    Thymidyl,
oder eine Modifikation davon ist, wobei diese Desoxynucleotidsequenz für Rinder-Präwachstumshormon codiert.

2. DNA-Transfervektor, der eine Desoxynucleotidsequenz umfaßt, die aus einem Plus-Strang mit der Sequenz besteht:

```
5' - GCC TTC CCA GCC ATG TCC TTG TCC GGC CTG
TTT GCC AAC GCT GTG CTC CGG GCT CAG CAC CTG CAC CAG CTG
GCT GCT GAC ACC TTC AAA GAG TTT GAG CCT ACC TAC ATC CCG
GAG GGA CAG AGA TAC TCC ATC CAG AAC ACC CAG GTT GCC TTC
TGC TTC TCC GAA ACC ATC CCG GCC CCC ACG GGC AAG AAT GAG
GCC CAG CAG AAA TCA GAC TTG GAG CTG CTT CGC ATC TCA CTG
CTC CTC ATC CAG TCG TGG CTT GGG CCC CTG CAG TTT CTC AGC
AGA GTC TTC ACC AAC AGC TTG GTG TTT GGC ACC TCG GAC CGT
GTC TAT GAG AAG CTG AAG GAC CTG GAG GAA GGC ATC TTG GCC
CTG ATG CGG GAG CTG GAA GAT GGC ACC CCC CGG GCT GGG CAG
ATC CTC AAG CAG ACC TAT GAC AAA TTT GAC ACA AAC ATG CGC
AGT GAC GAC GCG CTG CTC AAG AAC TAC GGT CTG CTC TCC TGC
TTC CGG AAG GAC CTG CAT AAG ACG GAG ACG TAC CTG AGG GTC
ATG AAG TGC CGC CGC TTC GGG GAG GCC AGC TGT GCC TTC TAG
- 3'
```

worin
A    Desoxyadenyl,
G    Desoxyguanyl,
C    Desoxycytosyl und
T    Thymidyl,

oder eine Modifikation davon ist, wobei diese Desoxynucleotidsequenz für ein Rinderwachstumshormon codiert.

3.  DNA-Transfervektor gemäß Anspruch 2, derart modifiziert, daß diese Desoxynucleotidsequenz aus einem Plus-Strang mit der Sequenz besteht:

```
5' - TTC CCA GCC ATG TCC TTG TCC GGC CTG TTT
GCC AAC GCT GTG CTC CGG GCT CAG CAC CTG CAC CAG CTG GCT
GCT GAC ACC TTC AAA GAG TTT GAG CGT ACC TAC ATC CCG GAG
GGA CAG AGA TAC TCC ATC CAG AAC ACC CAG GTT GCC TTC TGC
```

```
TTC TCC GAA ACC ATC CCG GCC CCC ACG GGC AAG AAT GAG GCC

CAG CAG AAA TCA GAC TTG GAG CTG CTT CGC ATC TCA CTG CTC.

CTC ATC CAG TCG TGG CTT GGG CCC CTG CAG TTT CTC AGC AGA

GTC TTC ACC AAC AGC TTG GTG TTT GGC ACC TCG GAC CGT GTC

TAT GAG AAG CTG AAG GAC CTG GAG GAA GGC ATC TTG GCC CTG

ATG CGG GAG CTG GAA GAT GGC ACC CCC CGG GCT GGG CAG ATC

CTC AAG CAG ACC TAT GAC AAA TTT GAC ACA AAC ATG CGC AGT

GAC GAC GCG CTG CTC AAG AAC TAC GGT CTG CTC TCC TGC TTC

CGG AAG GAC CTG CAT AAG ACG GAG ACG PAC CTG AGG GTC ATG

AAG TGC CGC CGC TTC GGG GAG GCC AGC TGT GCC TTC TAG - 3'
```

worin

A Desoxyadenyl,

G Desoxyguanyl,

C Desoxycytosyl und

T Thymidyl,

oder eine Modifikation davon ist.

4. Bakterium, das durch den Transfervektor gemäß irgendeinem der Ansprüche 1 bis 3 tranformiert ist.

5. Bakterium gemäß Anspruch 4, wobei das Bakterium Escherichia coli ist.

6. Bakterium gemäß Anspruch 5, wobei das Bakterium ausgewählt ist aus der Gruppe, die Escherichia coli χ 1776, HB101 oder RRI enthält.

7. Verfahren zur Herstellung eines DNA-Transfervektors zur Verwendung beim Erhalten und der Replikation einer Desoxynucleotidsequenz, die für ein Rinder-Präwachstumshormon codiert, das die Reaktion einer Desoxynucleotidsequenz, die für Rinder-Präwachstumshormon kodiert, mit einem durch Spalten eines Transfervektors mit einem Restriktionsenzym dargestellten DNA-Molekül umfaßt, wobei diese Desoxynucleotidsequenz aus einem Plus-Strang mit der Sequenz besteht:

5' - ATG ATG GCT GCA GGC CCC CGG ACC TCC CTG
CTC CTG GCT TTC GCC CTG CTC TGC CTG CCC TGG ACT CAG GTC
GTG GGC GCC TTC CCA GCC ATG TCC TTG TCC GGC CTG TTT GCC
AAC GCT GTG CTC CGG GCT CAG CAC CTG CAC CAG CTG GCT GCT
GAC ACC TTC AAA GAG TTT GAG CGT ACC TAC ATC CCG GAG GGA
CAG AGA TAC TCC ATC CAG AAC ACC CAG GTT GCC TTC TGC TTC
TCC GAA ACC ATC CCG GCC CCC ACG GGC AAG AAT GAG GCC CAG
CAG AAA TCA GAC TTG GAG CTG CTT CGC ATC TCA CTG CTC CTC
ATC CAG TCG TGG CTT GGG CCC CTG CAG TTT CTC AGC AGA GTC
TTC ACC AAC AGC TTG GTG TTT GGC ACC TCG GAC CGT GTC TAT
GAG AAG CTG AAG GAC CTG GAG GAA GGC ATC TTG GCC CTG ATG
CGG GAG CTG GAA GAT GGC ACC CCC CGG GCT GGG CAG ATC CTC
AAG CAG ACC TAT GAC AAA TTT GAC ACA AAC ATG CGC AGT GAC
GAC GCG CTG CTC AAG AAC TAC GGT CTG CTC TCC TGC TTC CGG
AAG GAC CTG CAT AAG ACG GAG ACG TAC CTG AGG GTC ATG AAG
TGC CGC CGC TTC GGG GAG GCC AGC TGT GCC TTC TAG - 3'

worin

A    Desoxyadenyl,
G    Desoxyguanly,
C    Desoxycytosyl und
T    Thymidyl,

oder eine Modifikation davon ist, wobei diese Desoxynucleotidsequenz für Rinder-Präwachstumshormon codiert.

8.  Verfahren zum Darstellen einer Desoxynucleotidsequenz zur Verwendung bei der Synthese von Rinderwachstumshormon oder einem wesentlichen Teil davon, gekennzeichnet durch Spalten einer cDNA, die für Rinder-Präwachstumshormon codiert, die mittels eines Vektors erhalten und repliziert worden ist, der gemäß dem Verfahren nach Anspruch 7 dargestellt worden ist mit dem Restriktionsenzym Hae II und Inkubation des abgespaltenen Produkts mit einem Enzym, das aus der Gruppe ausgewählt ist, die das Klenow-Fragment der DNA-Polymerase I, T4 DNA-Polymerase oder 3'-5'-Exonuclease enthält.

9.  Verfahren gemäß Anspruch 8, dadurch gekennzeichnet, daß die Inkubation mit diesem Klenow-Fragment in Gegenwart von dATP durchgeführt wird und das resultierende Produkt mit S1-Nuclease verdaut wird, wodurch eine Desoxynucleotidsequenz erhalten wird, die für die Aminosäuren 2-191 des Rinderwachstumshormons codiert.

10. Verfahren gemäß Anspruch 9, dadurch gekennzeichnet, daß die Desoxynucleotidsequenz, die für Rinderwachstumshormon codiert, aus einem Plus-Strang mit der Sequenz besteht:

```
5' - TTC CCA GCC ATG TCC TTG TCC GGC CTG TTT
GCC AAC GCT GTG CTC CGG GCT CAG CAC CTG CAC CAG CTG GCT
GCT GAC ACC TTC AAA GAG TTT GAG CGT ACC TAC ATC CCG GAG
GGA CAG AGA TAC TCC ATC CAG AAC ACC CAG GTT GCC TTC TGC
TTC TCC GAA ACC ATC CCG GCC CCC ACG GGC AAG AAT GAG GCC
CAG CAG AAA TCA GAC TTG GAG CTG CTT CGC ATC TCA CTG CTC
CTC ATC CAG TCG TGG CTT GGG CCC CTG CAG TTT CTC AGC AGA
GTC TTC ACC AAC AGC TTG GTG TTT GGC ACC TCG GAC CGT GTC
TAT GAG AAG CTG AAG GAC CTG GAG GAA GGC ATC TTG GCC CTG
ATG CGG GAG CTG GAA GAT GGC ACC CCC CGG GCT GGG CAG ATC
CTC AAG CAG ACC TAT GAC AAA TTT GAC ACA AAC ATG CGC AGT


GAC GAC GCG CTG CTC AAG AAC TAC GGT CTG CTC TCC TGC TTC·
CGG AAG GAC CTG CAT AAG ACG GAG ACG TAC CTG AGG GTC ATG
AAG TGC CGC CGC TTC GGG GAG GCC AGC TGT GCC TTC TAG - 3'
```

worin

| | |
|---|---|
| A | Desoxyadenyl, |
| G | Desoxyguanyl, |
| C | desoxycytosyl und |
| T | Thymidyl, |

oder eine Modifikation davon ist, wobei diese Desoxynucleotidsequenz für Rinderwachstumshormon codiert.

11. Verfahren gemäß Anspruch 8, dadurch gekennzeichnet, daß die Inkubation mit diesem Klenow-Fragment in Gegenwart von dATP, dGTP, dCTP und dTTP durchgeführt wird, das resultierende Produkt mit einem Enzym inkubiert wird, das aus der Gruppe ausgewählt ist, die dieses Klenow-Fragment, T4 DNA-Polymerase oder 3'-5'-Exonuclease in Gegenwart von dCTP enthält und das resultierende Produkt mit S1-Nuclease verdaut, wodurch eine Desoxynucleotidsequenz erhalten wird, die für die Aminosäuren 1-191 des Rinderwachstumshormons codiert.

12. Verfahren gemäß Anspruch 11, dadurch gekennzeichnet, daß die Desoxynucleotidsequenz, die für Rinderwachstumshormon codiert, aus einem Plus-Strang mit der Sequenz besteht:

```
5' - GCC TTC CCA GCC ATG TCC TTG TCC GGC CTG
TTT GCC AAC GCT GTG CTC CGG GCT CAG CAC CTG CAC CAG CTG
GCT GCT GAC ACC TTC AAA GAG TTT GAG CGT ACC TAC ATC CCG
GAG GGA CAG AGA TAC TCC ATC CAG AAC ACC CAG GTT GCC TTC
TGC TTC TCC GAA ACC ATC CCG GCC CCC ACG GGC AAG AAT GAG
GCC CAG CAG AAA TCA GAC TTG GAG CTG CTT CGC ATC TCA CTG
CTC CTC ATC CAG TCG TGG CTT GGG CCC CTG CAG TTT CTC AGC
```

```
AGA GTC TTC ACC AAC AGC TTG GTG TTT GGC ACC TCG GAC CGT
GTC TAT GAG AAG CTG AAG GAC CTG GAG GAA GGC ATC TTG GCC
CTG ATG CGG GAG CTG GAA GAT GGC ACC CCC CGG GCT GGG CAG
ATC CTC AAG CAG ACC TAT GAC AAA TTT GAC ACA AAC ATG CGC
AGT GAC GAC GCG CTG CTC AAG AAC TAC GGT CTG CTC TCC TGC
TTC CGG AAG GAC CTG CAT AAG ACG GAG ACG TAC CTG AGG GTC
ATG AAG TGC CGC CGC TTC GGG GAG GCC AGC TGT GCC TTC TAG
- 3'
```

worin

- A Desoxyadenyl,
- G Desoxyguanyl,
- C Desoxycytosyl und
- T Thymidyl,

oder eine Modifikation davon ist.

13. Verfahren gemäß Anspruch 7, dadurch gekennzeichnet, daß das DNA-Molekül, das durch Abtrennen eines Transfervektors mit einem Restriktionsenzym dargestellt worden ist, pBR322 ist, und das Restriktionsenzym Pst I ist.

14. Verfahren zur Herstellung eines DNA-Transfervektors zur Verwendung bei der Darstellung und Replikation einer Desoxynucleotidsequenz, die für Rinderwachstumshormon codiert, gekennzeichnet durch Reaktion einer Desoxynucleotidsequenz, die für Rinderwachstumshormon codiert, die gemäß irgendeinem der Ansprüche 1 bis 12 hergestellt worden ist, mit einem DNA-Molekül, das durch Abtrennen eines Transfervektors mit einem Restriktionsenzym dargestellt worden ist.

15. Verfahren zur Herstellung eines Expressionstransfervektors gemäß Anspruch 7 oder Anspruch 14 zur Verwendung bei der Expression einer Desoxynucleotidsequenz, die für Rinder-Präwachstumshormon oder Rinderwachstumshormon codiert, dadurch gekennzeichnet, daß das DNA-Molekül, das durch Abtrennen eines Transfervektors mit einem Restriktionsenzym hergestellt worden ist, an einem Punkt innerhalb eines expremierten Kontrollbereiches abgetrennt wird.

16. Verfahren zur Herstellung eines Fusionsproteins, das die Aminosäuresequenz von Rinder-Präwachstumshormon oder die Aminosäureseuqenz von Rinderwachstumshormon als ihr C-terminales Ende und einen procaryontischen Proteinabschnitt als ihr N-terminales Ende enthält, gekennzeichnet durch Inkubation eines Mikroorganismus, der durch einen Expressionstransfervektor transformiert ist, der eine Desoxynucleotidsequenz enthält, der für dieses Rinder-Präwachstumshormon oder für dieses Rinderwachstumshormon, das gemäß Anspruch 15 dargestellt worden ist, codiert.

17. Verfahren zur Synthetisierung von Rinderwachstumshormon, gekennzeichnet durch Inkubation eines durch einen Expressionstransfervektor transformierten Mikroorganismus, der eine Desoxynucleotidsequenz enthält, die für dieses Rinderwachstumshormon codiert, das gemäß irgendeinem der Ansprüche 8 bis 12 unter Bedingungen hergestellt worden ist, die zur Expression dieser Sequenz geeignet sind, die für Rinderwachstumshormon codiert und Reinigung des Rinderwachstumshormons aus dem Lysat oder Zellkulturmedium dieses Mikroorganismus.

18. Verfahren zur Synthetisierung von Rinderwachstumshormon oder Rinder-Präwachstumshormon, gekennzeichnet durch Inkubation eines durch einen Expressionstransfervektor transformierten Mikroorganismus, der eine DNA-Sequenz enthält, wie in jedem der Ansprüche 1 bis 3 dargestellt ist, unter Bedingungen, die zur Expression dieser Sequenz geeignet sind, die für Rinderwachstumshormon oder Rinder-Präwachstumshormon codiert und Reinigung des Rinderwachstumshormons oder Rinder-Präwachstumshormons aus dem Lysat oder Zellkulturmedium dieses Mikroorganismus.

```
                                    -26                                    -20
                                    met met ala ala gly pro arg thr ser leu leu leu ala phe ala leu
ACGGCTCAGGGTCCGTGACGCTCACCAGCT ATG ATG GCT GCA GGC CCC CGG ACC TCC CTG CTC CTG GCT TTC GCC CTG


-10                                                    1                                      10
leu cys leu pro trp thr gln val val gly ala phe pro ala met ser leu ser gly leu phe ala asn ala
CTC TGC CTG CCC TGG ACT CAG GTG GTG GGC GCC TTC CCA GCC ATG TCC TTG TCC GGC CTG TTT GCC AAC GCT


           20                                                    30
val leu arg ala gln his leu his gln leu ala ala asp thr phe lys glu phe glu arg thr tyr ile pro
GTG CTC CGG GCT CAG CAC CTG CAC CAG CTG GCT GCT GAC ACC TTC AAA GAG TTT GAG CGT ACC TAC ATC CCG


      40                                                    50                                      60
glu gly gln arg tyr ser ile gln asn thr gln val ala phe cys phe ser glu thr ile pro ala pro thr
GAG GGA CAG AGA TAC TCC ATC CAG AAC ACC CAG GTT GCC TTC TGC TTC TCC GAA ACC ATC CCG GCC CCC ACG


              70                                                    80
gly lys asn glu ala gln gln lys ser asp leu glu leu leu arg ile ser leu leu leu ile gln ser trp
GGC AAG AAT GAG GCC CAG CAG AAA TCA GAC TTG GAG CTG CTT CGC ATC TCA CTG CTC CTC ATC CAG TCG TGG


         90                                                    100                                     110
leu gly pro leu gln phe leu ser arg val phe thr asn ser leu val phe gly thr ser asp arg val tyr
CTT GGG CCC CTG CAG TTT CTC AGC AGA GTC TTC ACC AAC AGC TTG GTG TTT GGC ACC TCG GAC CGT GTC TAT


                                              120                                     130
glu lys leu lys asp leu glu glu gly ile leu ala leu met arg glu leu glu asp gly thr pro arg ala
GAG AAG CTG AAG GAC CTG GAG GAA GGC ATC TTG GCC CTG ATG CGG GAG CTG GAA GAT GGC ACC CCC CGG GCT


   140                                                    150
gly gln ile leu lys gln thr tyr asp lys phe asp thr asn met arg ser asp asp ala leu leu lys asn
GGG CAG ATC CTC AAG CAG ACC TAT GAC AAA TTT GAC ACA AAC ATG CGC AGT GAC GAC GCG CTG CTC AAG AAC


   160                                                    170                                     180
tyr gly leu leu ser cys phe arg lys asp leu his lys thr glu thr tyr leu arg val met lys cys arg
TAC GGT CTG CTC TCC TGC TTC CGG AAG GAC CTG CAT AAG ACG GAG ACG TAC CTG AGG GTC ATG AAG TGC CGC


                  190 191
arg phe gly glu ala ser cys ala phe AM
CGC TTC GGG GAG GCC AGC TGT GCC TTC TAG TTGCCAGCCATCTGTTGTTTGCCCCTCCCCCGTGCCTTCCTTGACCCTGGAAGG


TGCCACTCCCACTGTCCTTTCCTAATAAAATGAGGAAATTGCATCGC[poly A]        FIG. I
```

EP 0 047 600 B1

(a) (b) (c)

(A)

(B)
(C)

FIG.2